Europäisches Patentamt

European Patent Office

Office européen des brevets

(19)

(11) EP 0 720 595 B1

(12)

# FASCICULE DE BREVET EUROPEEN

(45) Date de publication et mention
de la délivrance du brevet:
**23.12.1998 Bulletin 1998/52**

(51) Int Cl.[6]: **C07B 57/00**

(21) Numéro de dépôt: **94928427.7**

(86) Numéro de dépôt international:
**PCT/FR94/01107**

(22) Date de dépôt: **22.09.1994**

(87) Numéro de publication internationale:
**WO 95/08522 (30.03.1995 Gazette 1995/14)**

(54) **PROCEDE DE DEDOUBLEMENT DE DEUX ANTIPODES OPTIQUES PAR CRISTALLISATION**

VERFAHREN ZUR OPTISCHEN SPALTUNG VON ANTIPODEN DURCH KRISTALLISIERUNG

METHOD OF RESOLUTION OF TWO ENANTIOMERS BY CRYSTALLIZATION

(84) Etats contractants désignés:
**AT BE CH DE DK ES FR GB GR IE IT LI LU MC NL PT SE**

(30) Priorité: **23.09.1993 FR 9311354**

(43) Date de publication de la demande:
**10.07.1996 Bulletin 1996/28**

(73) Titulaire: **UNIVERSITE DE ROUEN**
**F-76821 Mont-Saint-Angan Cédex (FR)**

(72) Inventeurs:
• **COQUEREL, Gérard**
**F-76960 Notre-Dame-de-Bondeville (FR)**
• **PETIT, Marie-Noelle**
**F-76960 Mont-Saint-Aignan (FR)**

• **BOUAZIZ, Roger**
**F-75116 Paris (FR)**

(74) Mandataire: **Pernez, Helga**
**Breese-Majerowicz,**
**3, avenue de l'Opéra**
**75001 Paris (FR)**

(56) Documents cités:
**FR-A- 1 456 627          GB-A- 1 197 809**

• **TETRAHEDRON LETTERS, vol. 31, no. 15, 1990, Oxford (GB); G. COQUEREL et al., pp. 2143-2144**
• **CHEMICAL REVIEWS, vol. 80, no. 3, 1980, Easton, PA (US); A. COLLET et al., pp. 215-230**
• **BULLETIN DE LA SOCIETE CHIMIQUE DE FRANCE, 1956, Paris (FR); G. AMIARD, p. 447**

**Description**

La présente invention concerne le domaine de la séparation de deux antipodes optiques d'une espèce chimique par cristallisation préférentielle. Cette méthode repose sur la cristallisation alternée de deux composés chiraux dénommés R et S, réalisant un conglomérat dans un solvant A et pour une gamme donnée de température ΔT. Ceci signifie que dans cette gamme de température, tout mélange, en équilibre thermodynamique des deux antipodes avec la solution, est constitué de deux types de cristaux ne contenant chacun que des molécules de même configuration, incorporant ou non, des molécules de solvant (solvates). L'existence d'un tel conglomérat, sans miscibilité à l'état solide, sera implicitement admise dans ce qui suit, au moins dans l'intervalle de température ΔT et pour le solvant A.

La technique de cristallisation préférentielle est largement mise en oeuvre dans les laboratoires et dans l'industrie grâce aux principaux avantages qu'elle apporte :

- elle évite l'utilisation d'un agent chiral intermédiaire, onéreux, dont la récupération ultérieure implique des pertes rarement inférieures à 10 % (De Min, M., Levy, G., et Micheau, J. C. (1988) J. Chim. Phys. 85, 603-19);
- les deux antipodes sont obtenus directement, contrairement à la méthode mettant en oeuvre le dédoublement classique par formation de sels diastéréoisomères;
- le rendement est théoriquement quantitatif par suite de recyclages successifs;
- la purification des cristaux d'énantiomères bruts obtenus est aisée.

Deux classes de facteurs influencent la cristallisation des antipodes optiques, d'une part, les paramètres liés aux équilibres hétérogènes ternaires et d'autre part, les facteurs intervenant sur la cinétique de cristallisation.

Les paramètres liés aux équilibres hétérogènes ternaires comprennent:

- les positions des nappes de cristallisation des espèces solides qui se déposent à chaque température, et plus particulièrement, les valeurs des solubilités des phases stables et métastables, du mélange racémique $s(\pm)$ et des antipodes $s(+) = s(-)$, en fonction de la température, et le rapport des solubilités $\alpha = s(\pm) / s(+)$ ;
- l'étendue des domaines stables et métastables de solutions solides, de racémate, de solvate racémique, de solvates actifs et des variétés polymorphiques des solides cristallisés.

Les facteurs intervenant sur la cinétique de cristallisation comprennent:

- des facteurs internes aux cristaux, en relation avec les liaisons entre les molécules, qui sont non modifiables par l'expérimenteur;
- des facteurs externes qui sont modifiables par l'expérimentateur; il s'agit de la nature du solvant, de la nature et de la concentration des impuretés, de la sursaturation acquise en fonction du temps, de l'intervalle de température ΔT, de la vitesse et du mode d'agitation, de la masse et de la granulométrie des germes, de l'effet de paroi, etc....

Ces deux classes de facteurs influencent directement le rendement, la pureté des phases obtenues et le déroulement des opérations de séparation. La faisabilité de la filtration sera ainsi dépendante du spectre granulométrique et de l'habitus des cristaux, de la viscosité de la suspension, de la tension de vapeur du solvant, des sursaturations acquises par chacun des antipodes et de la présence éventuelle d'un racémate vrai à caractère métastable. Ces choix pourront également intervenir sur la cinétique de racémisation des antipodes ou de dégradation de la molécule.

Pour chaque ensemble constitué du couple d'antipodes (R et S) et du solvant (A), les facteurs intervenant sur la cinétique sont un cas d'espèce.

La présente invention vise à améliorer les procédés de cristallisation préférentielle de l'Art antérieur à partir d'une meilleure connaissance des équilibres hétérogènes ternaires réalisés à partir du solvant A et des antipodes optiques R et S. En effet, la connaissance des équilibres hétérogènes fournit des données mises à profit dans le procédé de l'invention pour réaliser un dédoublement plus efficace et rationnel des deux antipodes optiques par cristallisation préférentielle (ou balancement).

Plus particulièrement, le procédé de l'invention permet d'optimiser et de généraliser un procédé du type de celui décrit dans le Brevet Français n° 1 456 627 concernant la résolution de l'acide glutamique et de ses sels.

Le procédé de l'invention repose sur une modification de portée générale des procédés classiques désignés ci-après SIPC pour "Seeded isothermal preferential crystallization" et des variantes polythermiques, grâce à l'exploitation d'informations contenues dans la représentation des équilibres ternaires : antipode R - antipode S - solvant A.

Le procédé de l'invention consiste :

a) à réaliser un ensemble composé du mélange racémique de cristaux sous forme de conglomérat, du premier énantiomère et de solvant, dont le point figuratif E, défini par les variables concentration et température $T_B$, se situe dans le domaine biphasé du premier énantiomère en excès, et en équilibre avec sa solution saturée;

b) à appliquer une loi de programmation de l'abaissement de température au mélange biphasé préparé à l'étape précédente, telle que les liqueurs-mères gardent une faible sursaturation qui privilégie la croissance de l'énantiomère présent sous forme de cristaux, tout en interdisant la nucléation spontanée du second énantiomère présent dans la solution;

c) à adapter pendant toute la durée de la croissance cristalline de l'étape précédente une vitesse d'agitation légèrement croissante en fonction du temps de façon à ce que celle-ci soit à tout moment, suffisamment lente pour favoriser une croissance du premier énantiomère en évitant de générer des forces de striction trop importantes provoquant une nucléation non maîtrisée, et suffisamment rapide pour réaliser une suspension homogène et un renouvellement rapide de la liqueur-mère autour de chaque cristallite du premier énantiomère;

d) à récolter les cristaux du premier énantiomère;

e) à additionner aux liqueurs-mères résultant de la récolte réalisée à l'étape précédente, le mélange racémique de cristaux sous forme de conglomérat, et à porter le nouvel ensemble à un palier de température $T_B$ pendant la durée nécessaire à l'obtention de l'équilibre thermodynamique de sorte que le point figuratif E' soit symétrique de E par rapport au plan des mélanges racémiques du système solvant, antipode (-), antipode (+), ledit point E' se situant dans le domaine biphasé du second énantiomère en excès et en équilibre avec sa solution saturée;

f) à appliquer la même loi de programmation de l'abaissement de température qu'à l'étape (b), au mélange biphasé préparé à l'étape précédente contenant le second énantiomère, de sorte que les liqueurs-mères gardent une faible sursaturation pendant la cristallisation afin de privilégier la croissance de l'énantiomère présent sous forme de cristaux tout en interdisant la nucléation spontanée du premier énantiomère présent dans la solution;

g) à adapter pendant toute la durée de la croissance cristalline de l'étape précédente, une vitesse d'agitation légèrement croissante en fonction du temps de façon à ce que celle-ci soit, à tout moment, suffisamment lente pour favoriser la croissance du second énantiomère en évitant de générer des forces de striction trop importantes provoquant une nucléation non maîtrisée, et suffisamment rapide pour obtenir une suspension homogène et un renouvellement rapide de la liqueur-mère autour de chaque cristallite;

h) à récolter les cristaux du second énantiomère;

i) à additionner aux liqueurs-mères résultant de la récolte cristalline réalisée à l'étape précédente, le mélange racémique de cristaux sous forme de conglomérat, pour obtenir un ensemble dont la composition est identique à celle de l'ensemble E initial;

j) à répéter les étapes (a), (b), (c), (d), (e), (f), (g), (h) et (i) pour obtenir successivement l'un puis l'autre des deux énantiomères.

L'optimisation et la reproductibilité du procédé de l'invention sont réalisées grâce à la détermination des paramètres thermodynamiques et cinétiques.

Ainsi, à l'étape (a) du procédé de l'invention, le choix du ou des solvants et de la gamme de température de travail sont définis de façon à avoir simultanément :

- des antipodes qui réalisent un conglomérat et dont l'éventuel racémate est métastable dans la gamme de température de travail;
- des liqueurs suffisamment concentrées mais de faible viscosité et de faible tension de vapeur;
- une absence de solvolyse et de racémisation;
- une stabilité des solvates si ceux-ci sont présents à l'équilibre et s'il s'agit d'énantiomères dédoublables.

Aux étapes (a) et (e) du procédé de l'invention, la température $T_B$ est supérieure à la température $T_L$ d'homogénéisation de la quantité de mélange racémique contenue dans la suspension initiale, et en ce que, à partir de la courbe de variation de $T_{HOMO}$ en fonction de l'excès énantiomérique et pour une concentration constante en mélange racémique $X_L$, ladite température $T_B$ est définie de façon à ce que la masse de fins cristaux du premier énantiomère des étapes (a) et (i) et du second énantiomère de l'étape (e), en équilibre avec leur solution saturée, représente au maximum 50 % et de préférence entre environ 25 et 40 % de la récolte attendue

Aux étapes (b) et (f) du procédé de l'invention, la loi de programmation de l'abaissement de température $T_B$ à $T_F$, adaptée au montage expérimental, est définie de façon :

- à obtenir une faible sursaturation pendant toute la durée de la cristallisation de l'énantiomère présent sous forme de cristaux au début de chaque cycle, cette faible sursaturation provoquant une croissance et une nucléation secondaires douces;
- à atteindre à $T_F$ le maximum de sursaturation de l'autre énantiomère sans nucléation primaire,
- à obtenir une récolte en cristaux aux étapes (d) et (h) qui, après addition de mélange racémique et compensation aux étapes (e) et (i), permet la cyclicité des opérations.

En effet, chaque montage expérimental influe sur les capacités de sursaturation des mélanges utilisés et l'efficacité de l'agitation, et, en conséquence, la loi de programmation du refroidissement est adaptée aux circonstances dans lesquelles le procédé est mis en oeuvre. Pour autant la température TB, les solubilités du mélange racémique en fonction de la température, la courbe $T_{HOMO}$ en fonction de l'excès énantiomérique pour une concentration constante en mélange racémique $X_L$, sont elles totalement indépendantes du montage expérimental.

La loi de programmation de l'abaissement de température, qui est la fonction reliant la température au temps, est déterminée pour sa partie de $T_L$ à $T_F$ par refroidissement de la solution de concentration $X_L$ de $T_L$ + 1°C à $T_F$, $T_F$ étant inférieur à $T_L$- $(T_{HOMO}$- $T_L)$, afin d'obtenir une solution sursaturée sans nucléation primaire tout en permettant une récolte double de l'excès énantiomérique initial, et en ce que ladite loi de programmation de l'abaissement de température est déterminée pour sa partie de $T_B$ à $T_L$ par extrapolation de cette même loi déterminée de $T_L$ + 1°C à $T_F$.

Le procédé de l'invention présente d'autres caractéristiques avantageuses, seules ou en combinaison, telles que :

- aux étapes (a) et (i), la masse de fins cristaux du premier énantiomère en équilibre avec sa solution saturée représente entre, environ, 25 et 40 % de la récolte attendue, 50 % représentant une limite maximale;
- à l'étape (e), la masse de fins cristaux du second énantiomère en équilibre avec sa solution saturée représente entre, environ, 25 et 40 % de la récolte attendue, 50 % représentant une limite maximale;
- aux étapes (b) et (f), la thermicité accompagnant le dépôt du premier énantiomère et du second énantiomère est intégrée dans la loi de programmation de l'abaissement de température;
- aux étapes (e) et (i), on effectue des compensations en solvant;
- aux étapes (a) , (e) et (i), les fins cristaux du mélange racémique sous forme de conglomérat qui sont ajoutés, ont subi avant d'être introduits un traitement préalable accélérant l'étape de dissolution, tel qu'un broyage et un tamisage, un traitement par des ondes ultra-sonores, une lyophilisation partielle; ces traitements ayant également pour but de fournir de fins cristaux propres à générer une surface de croissance cristalline élevée;
- aux étapes (a), (e) et (i), comportant une dissolution, la vitesse d'agitation est élevée par rapport aux étapes (c) et (g).

Outre les données des équilibres hétérogènes requises pour mettre en oeuvre le procédé de l'invention, les opérations restent également soumises à des contraintes cinétiques ajustables, notamment la loi de refroidissement, qui sont, pour chaque ensemble solvant, énantiomères, un cas d'espèce.

Le procédé de l'invention présente plusieurs avantages par rapport aux méthodes de l'Art antérieur :

- absence d'introduction de germes,
- obtention d'une meilleure récolte en cristaux,
- obtention d'une meilleure pureté des cristaux,
- obtention d'une meilleure reproductibilité des opérations,
- possibilité d'entreprendre le dédoublement sans avoir recours au préalable, à la séparation et à la purification des deux énantiomères,
- gain de temps à chaque cycle d'opération.

Les avantages cités ci-dessus permettent également d'envisager une plus grande facilité d'automatisation du procédé de l'invention par rapport aux méthodes de l'Art antérieur.

Une description du système ternaire A (solvant), R et S (antipodes optiques), à l'équilibre est nécessaire avant de comparer le procédé classique SIPC, d'une part avec ses variantes, et d'autre part avec le procédé de l'invention, désignée ci-après AS3PC pour "Auto-seeded programmed polythermic preferential crystallization".

Cette description comprendra aussi des exemples non limitatifs destinés à illustrer d'autres caractéristiques et avantages du procédé de l'invention.

A titre d'exemples de mise en oeuvre du procédé de l'invention, seront développés dans la description qui suit, le dédoublement des deux énantiomères optiques : du 3-5-dinitrobenzoate-1-pényléthanol, du tartrate double de sodium et d'ammonium tétrahydraté, du chlorhydrate de l'acide glutamique, de la thréonine, de la 5-méthyl-5-phényl-hydantoïne, de la 5-méthyl,5-(4 méthyl phényl)-hydantoïne, de la 5-éthyl-5-phényl-hydantoïne, de la 5-méthyl, 5-(4-chlorophényl)-hydantoïne, du thréitol, selon un montage expérimental particulier.

Il sera fait référence dans ce qui suit aux dessins annexés sur lesquels :

- La figure 1 est une représentation en perspective du système ternaire solvant A - antipode R - antipode S, en fonction de la température ainsi que des nappes de cristallisation de chaque constituant et des compositions des solutions doublement saturées (courbes monovariantes) ; sur cette figure sont également représentées les isothermes aux températures $T_D$ et à $T_F$ et le plan d'eutexie ternaire à la température $T_\varepsilon$ et renfermant quatre phases.
- La figure 2 est une projection, sur le plan des concentrations, des équilibres à $T_D$ et $T_F$; ainsi qu'une représentation de la trace de la coupe isoplèthe RY, sur laquelle le point E matérialise la composition du mélange initial légèrement enrichi en antipode R et devant déposer ce même antipode.

- La figure 3 est la coupe verticale isoplèthe RY de la figure 2 contenant les points composition de l'antipode en excès et celui de la solution initiale E, sur laquelle est représenté le cheminement, à l'équilibre et au refroidissement, du point-solution pour un mélange de composition $X_E$ (en trait gras). Pour $T < T_L$, le point-solution n'appartient plus à cette coupe.
- La figure 4 est une projection sur le plan des concentrations du cheminement du point-solution (en trait gras) lors du dédoublement alterné par entraînement isotherme à la température $T_F$ et ensemencé, selon la méthode SIPC.
- La figure 5 est la coupe verticale isoplèthe contenant la droite RY de la figure 4 et illustrant le cheminement du point-solution (en trait gras) de E à F lors de l'entraînement isotherme (à $T_F$) et ensemencé, selon la méthode SIPC.
- La figure 6 est une projection sur le plan des concentrations du cheminement du point-solution (en trait gras) lors du dédoublement par le procédé de l'invention polythermique programmé et auto-ensemencé (AS3PC).
- La figure 7 est la coupe verticale isoplèthe contenant la droite RY de la figure 6 et illustrant le cheminement du point-solution (en trait gras) de $S_E$ à F lors du dédoublement par le procédé de l'invention polythermique programmé et auto-ensemencé (AS3PC).
- La figure 8 est une projection sur le plan des concentrations du cheminement du point-solution (en trait gras) lors du dédoublement par le procédé de l'invention polythermique programmé et auto-ensemencé (AS3PC) et vérifiant la relation s(±) < 2 - α.

Toutes les coupes isothermes et isoplèthes représentées dans ces figures possèdent des variables compositions exprimées en fractions massiques.

I - <u>LES EQUILIBRES HETEROGENES TERNAIRES : ANTIPODES R ET S, ET SOLVANT A.</u>

Par exemple, l'ouvrage de J. E. Ricci (Ed. Dover Publication Inc. New York (1966) "The Phase Rule and Heterogeneous Equilibrium") traite du cas général des équilibres hétérogènes dans les systèmes ternaires. La description ci-après sera limitée aux aspects particuliers du système ternaire : A (solvant achiral), R et S (énantiomères non racémisables dans le domaine de température exploité), nécessaires à la compréhension des différents procédés de cristallisation préférentielle.

Afin de mettre en évidence le rôle particulier du solvant, ce système ternaire sera représenté à partir d'un prisme droit à section triangle rectangle isocèle, sur lequel la température est portée sur un axe perpendiculaire au plan des concentrations.

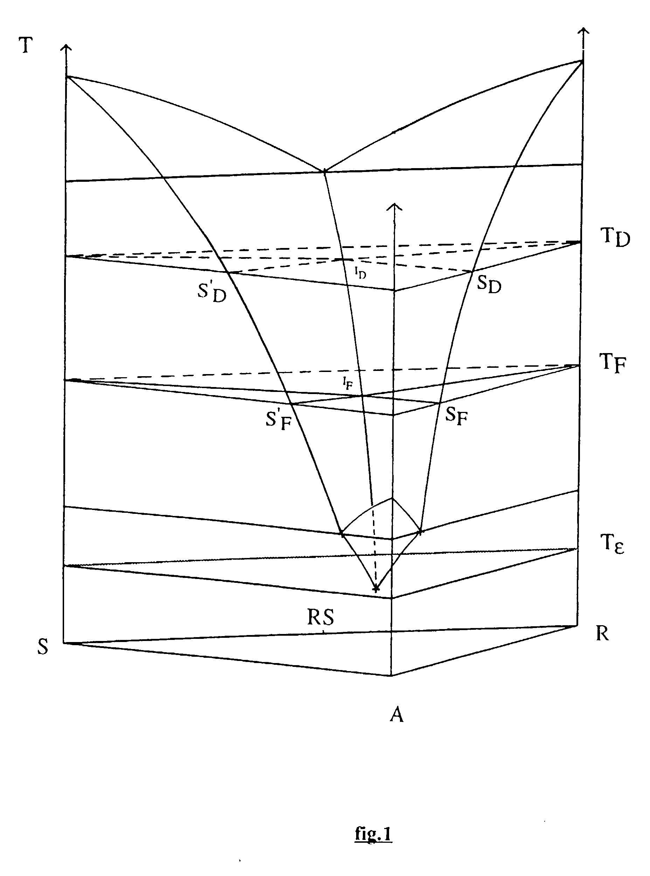

L'identité des variables thermodynamiques des deux énantiomères : Tf, ΔHf, solubilité dans un solvant achiral, etc..., fait que la représentation des domaines est symétrique par rapport au plan vertical A-RS-T, rassemblant les mélanges optiquement inactifs, de la figure 1. Afin de faciliter une première description de ce système, les simplificaions suivantes ont été adoptées:

- les seules phases qui cristallisent sont les constituants purs dans un arrangement donné (absence de racémate, de solvate et de polymorphisme pour les antipodes);
- la miscibilité entre les constituants indépendants est nulle à l'état solide;
- le solvant possède un point de fusion nettement inférieur à celui des antipodes;
- dans le domaine de température exploité, la solubilité d'un antipode n'est pas influencée par la présence du second dans la solution (loi de Meyerhoffer respectée), ce qui se traduit par une valeur du rapport α = 2).

1 - <u>Représentation des équilibres ternaires en fonction de la température.</u>

La figure 1 permet de visualiser les domaines de phases suivants :

- le domaine monophasé de la solution diluée (ϕ = 1);
- les trois nappes de cristallisation des constituants délimitant les domaines biphasés (ϕ = 2). La surface de dépôt du solvant est confinée au voisinage de A, car le point de fusion de ce constituant est nettement plus bas que celui des autres constituants, conformément aux conditions mentionnées précédemment;
- les trois courbes monovariantes (ϕ = 3) ou vallées eutectiques issues des points eutectiques binaires;
- l'invariant eutectique ternaire à TE (ϕ = 4), au-dessous duquel les trois constituants sont cristallisés.

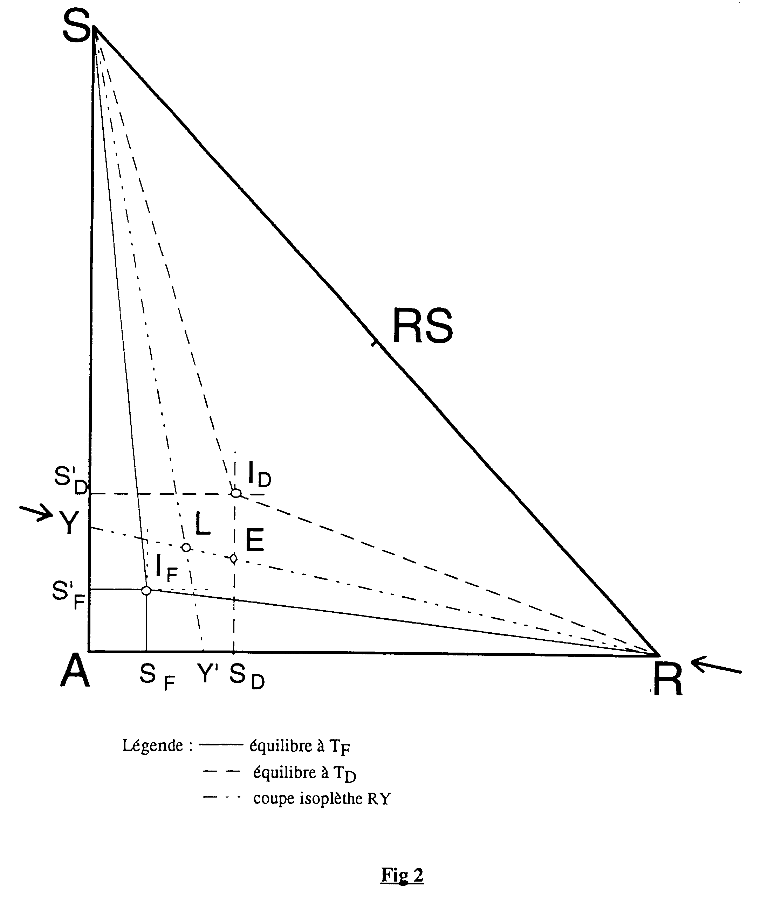

La figure 2 représente de façon superposée deux coupes isothermes à $T_D$ et $T_F$ du ternaire visualisé à la figure 1. A chaque température la coupe est composée de quatre domaines comme il est détaillé dans le tableau ci-dessous.

| Température | Limite du domaine | Nature des phases à l'équilibre | Nombre de phase à l'équilibre |
|---|---|---|---|
| $T_D$ | A-$_{SD}$-I$_D$-$_{S'D}$ | solution diluée | 1 |
| $T_D$ | R-$_{SD}$-I$_D$ | solution + cristaux de R | 2 |
| $T_D$ | S-$_{S'D}$-I$_D$ | solution + cristaux de S | 2 |
| $T_D$ | I$_D$-R-S | solution + cristaux de R et S | 3 |
| $T_F$ | A-$_{SF}$-I$_F$-$_{S'F}$ | solution diluée | 1 |
| $T_F$ | R-$_{SF}$-I$_F$ | solution + cristaux de R | 2 |
| $T_F$ | S-$_{S'F}$-I$_F$ | solution + cristaux de S | 2 |
| $T_F$ | I$_F$-R-S | solution + cristaux de R et S | 3 |

2 - Coupe isoplèthe RYT.

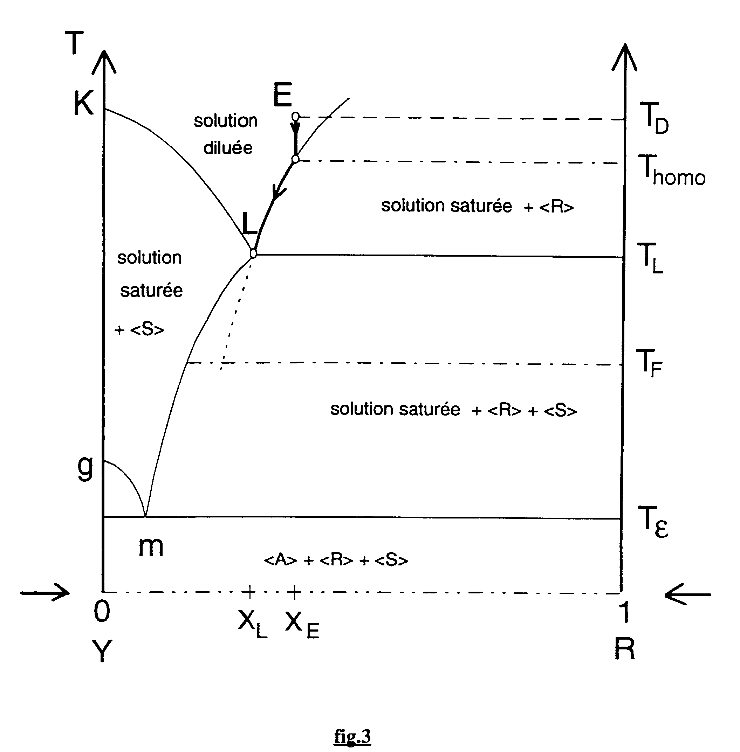

La figure 3 représente la coupe isoplèthe R-Y-T qui est fondamentale dans la compréhension de la cristallisation conduite par refroidissement de solutions ternaires, en quasi équilibre thermodynamique. Cette même coupe est également nécessaire pour le suivi des procédés hors équilibre, SIPC, variantes et AS3P3. Ce plan est le lieu géométrique des points vérifiant la relation :

$$X_A / X_S = (1-Y) / Y = \text{constante,}$$

avec $X_A$ et $X_S$ donnant les fractions massiques en solvant et en antipodes S.
A partir de la figure 3, on distingue :

- Le domaine monophasé de la solution ternaire.
- Le liquidus de l'antipode R, cette courbe représente l'intersection du plan R-Y de la figure 2 avec la nappe de cristallisation de ce constituant. Cette courbe d'équilibre stable prend naissance à la fusion de l'antipode R (non représentée) et se trouve limitée vers les basses températures par le point L, appartenant à la vallée eutectique ternaire des mélanges racémiques. Cette dernière courbe et la trace du conoïde à $T_L$ (segment horizontal à $T_L$) délimitent le domaine biphasé : solution saturée plus cristaux de R; elle se prolonge dans le domaine triphasé sous-jacent, par une courbe de solubilité à caractère métastable du même antipode R (en traits discontinus).
- Le domaine triphasé : cristaux de R et de S, plus solution saturée. Ce domaine est limité en haut par la trace horizontale du conoïde de R, vers le bas par la trace du plan invariant eutectique ternaire, à gauche par la trace Lm de l'un des conoïdes relatif à l'antipode S.
- La trace KL de la nappe de cristallisation de l'antipode S qui limite dans la partie supérieure le domaine biphasé : solution saturée plus cristaux de S. Ce domaine est limité dans sa partie inférieure par les traces des deux conoïdes de S : gm et Lm. La localisation de cette seconde trace Lm du conoïde de S par rapport à la courbe de solubilité métastable de R prolongement de EL sera discutée plus loin à propos de la position relative de F1 et F en fonction du rapport des solubilités $\alpha$.
- L'invariant ternaire à la température T$\epsilon$ au-dessous duquel se trouvent les trois constituants cristallisés A, R et S.

II - EVOLUTION AU REFROIDISSEMENT ET EN QUASI EQUILIBRE THERMODYNAMIQUE DE SOLUTIONS TERNAIRES PRESENTANT UN FAIBLE EXCES ENANTIOMERIQUE.

Il est considéré dans ce qui suit que le point ensemble du système (i.e. le point représentatif de la composition globale du mélange) se situe sur la verticale passant par le point E des figures 2 et 3, sa position précise est définie par sa température (ou cote) T. Seul l'intervalle de température suivant est envisagé :

- $T_D$: température pour laquelle le mélange de départ est une solution homogène, et,
- $T_F$: température de fin de cristallisation et filtration, située dans le domaine triphasé.

Cette composition globale E correspond à une solution racémique légèrement enrichie par une masse M d'antipode R et réalisant une masse totale Mt (l'excès énantiomérique R - S / R + S est généralement compris entre 4 et 9 %). Les conditions d'équilibre sont obtenues par un refroidissement très lent et par un ensemencement en phase(s) solide(s) dès que le point ensemble E figuratif du mélange parvient dans un domaine où cette (ces) phase(s) est (sont) présente(s) à l'équilibre.

A la température de départ $T_D$, la solution est homogène. Au refroidissement on observe successivement :

- La cristallisation de l'antipode R seul, de $T_{HOMO}$ jusqu'à $T_L$, simultanément le point solution se déplace sur la courbe de solubilité de l'antipode R à savoir du point E à la cote $T_{HOMO}$, au point L à l'intérieur de la coupe isoplèthe R-Y. Au point L, la masse M de cristaux R en équilibre avec la solution saturée est donnée par Mt ($X_E - X_L / 1 - X_L$) = M et correspond à l'excès énantiomérique présent dans la solution initiale (figure 3); les abscisses des points L, E et R correspondent aux compositions $X_L$, $X_E$ et 1 (figure 3).

- A partir de $T_L$, le point solution évolue de L vers $I_F$ sur la courbe monovariante contenant les solutions de composition racémique, représentée à la figure 2, sortant ainsi de la coupe isoplèthe R-Y de la figure 3; les cristaux de R et de S se déposent alors simultanément et en égale quantité.

Le dédoublement ne peut pas être réalisé dans des conditions d'équilibre pour des températures inférieures à $T_L$.

III - <u>EVOLUTION DU POINT SOLUTION LORS DU DEDOUBLEMENT PAR ENTRAINEMENT CONVENTIONNEL. CONFORMEMENT AU PROCEDE SIPC.</u>

1 - <u>Cristallisation du premier antipode en excès.</u>

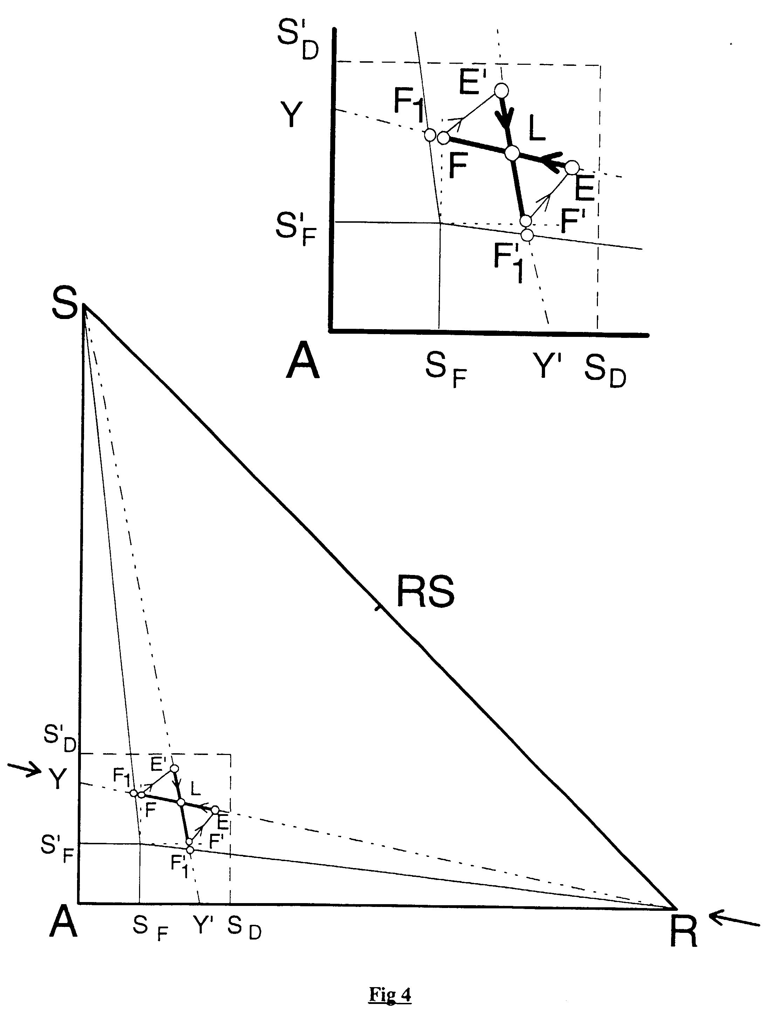

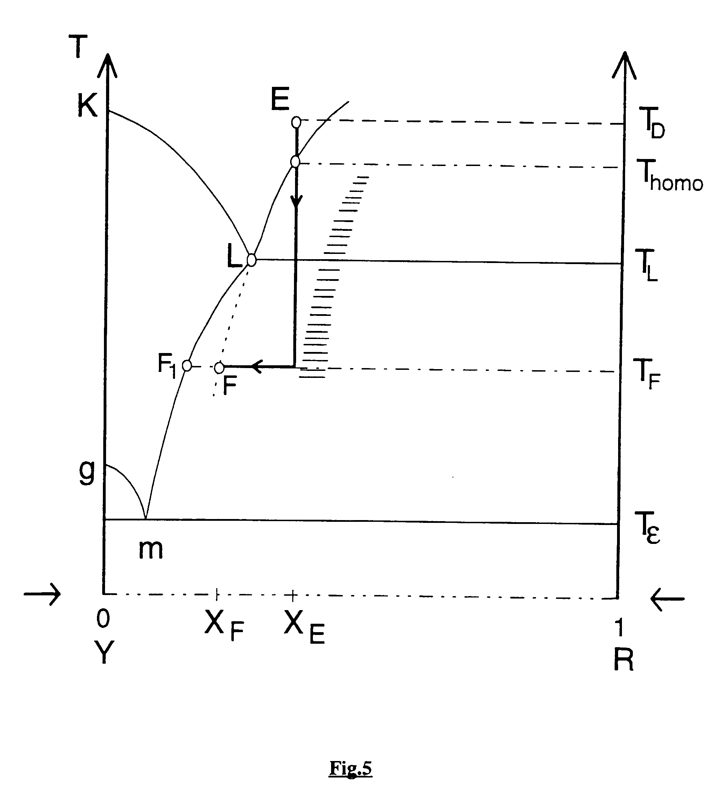

La solution E précédente est homogénéisée à la température $T_D$ (figures 4 et 5). Afin de la rendre sursaturée, elle est refroidie rapidement à la température $T_F$ sans qu'aucune cristallisation n'apparaisse. Cette solution, hors équilibre thermodynamique, est ensuite ensemencée par des germes d'antipode R très purs de même chiralité que celle de l'antipode en excès. La cristallisation isotherme de l'antipode R s'établit et le point représentatif de la solution évolue à l'intérieur de la coupe R-Y-T de E à la cote $T_F$ avec lequel il est d'abord confondu, jusqu'en F où l'on procède rapidement à une filtration. La masse d'antipode R récupérée est de 2M ou encore égale à Mt($X_E - X_F / 1 - X_F$).

2 - <u>Cristallisation du second antipode, cyclicité des opérations.</u>

L'opération fondamentale précédente a donc créé une solution F enrichie en antipode S. En ajoutant une masse 2M de mélange racémique (égale à celle de l'antipode récupéré) et en chauffant ce mélange à la température $T_D$, on obtient une solution homogène E' symétrique de E par rapport au plan vertical A-(RS)-T. Le processus permettant d'obtenir une masse 2M d'antipode S sera lui aussi représenté par un cheminement symétrique du précédent par rapport à ce plan médian. On procède donc en séquence aux opérations suivantes:

- la solution E' homogène à la température $T_D$ est d'abord refroidie à $T_F$, puis,
- ensemencée en germes très purs d'antipode S; la croissance de cet antipode déplace le point représentatif de la solution sur le segment horizontal E'F'(à la cote $T_F$);
- quand le point solution est confondu avec F', la solution est filtrée et fournit une masse 2M d'antipode S;
- après un nouvel ajout d'une masse 2M de mélange racémique et un nouveau chauffage à $T_D$, on obtient à nouveau une solution homogène dont le point représentatif est confondu avec le point initial E à la cote $T_D$;
- la suite du processus revient simplement à reproduire ce cycle d'opérations.

3 - <u>Variantes du procédé SIPC.</u>

La littérature (Amiard, G. (1956) Bull. Soc. Chim. Fr. 447 ; Collet, A., Brienne, M. J., Jacques, J. (1980) Chemical reviews 80, 3, 215-30 ; Noguchi Institute (1968) brevet GB 1 197 809) repose sur le schéma général précédent; les principales modifications apparues dans la littérature sont classées de la façon suivante :

a) <u>Nucléation primaire spontanée de l'antipode en excès.</u>

Lors du dédoublement de la (±) thréonine (Amiard, G. (1956) Bull. Soc. Chim. Fr. 447), la nucléation primaire de l'antipode en excès intervient spontanément au sein de la solution homogène sursaturée. Cette nucléation primaire se produit alors que le point E figurant la composition de l'ensemble se situe dans le domaine triphasé et que la solution n'est pas agitée (Collet, A., Brienne, M. J., Jacques, J. (1980) Chemical reviews 80, 3, 215-30) . Les résultats irréguliers auxquels conduit cette méthode seront analysés en comparaison avec ceux du procédé AS3PC.

b) Ensemencement pendant le refroidissement.

Ce protocole est le plus fréquemment rencontré dans la littérature (Noguchi Institute (1968) brevet GB 1 197 809) quand le procédé diffère de SIPC. Parmi les procédures citées des différences apparaissent; toutefois on peut dégager les grandes lignes communes suivantes :

- refroidissement de la solution homogène de $T_D$ à une température inférieure à $T_L$ mais supérieure à $T_F$;
- ensemencement, par des germes de même chiralité que celle de l'antipode en excès, de la solution homogène sursaturée située dans le domaine triphasé;
- refroidissement jusqu'à $T_F$. Dans certains cas, cette dernière étape est contrôlée par une programmation précise de la température (Noguchi Institute (1968) brevet GB 1 197 809).

On regroupera ces protocoles sous le même terme "S3PC" pour "Seeded polythermic programmed preferential crystallization" bien que la programmation de la température soit inexistante ou limitée à la deuxième phase du refroidissement. Les résultats obtenus lors du dédoublement du (±) chlorhydrate de l'acide glutamique par la méthode S3PC (Noguchi Institute (1968) brevet GB 1 197 809) et AS3PC seront comparés.

IV - EVOLUTION DU POINT SOLUTION LORS DU DEDOUBLEMENT PAR ENTRAINEMENT PROGRAMME ET AUTO-ENSEMENCE SELON LE PROCEDE DE L'INVENTION AS3PC.

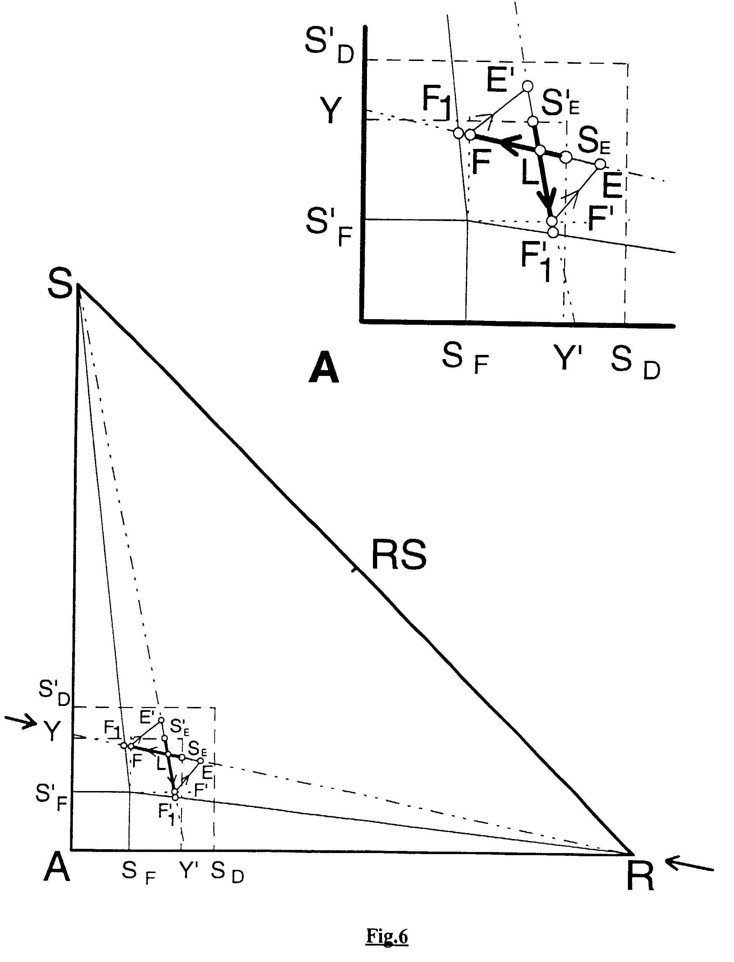

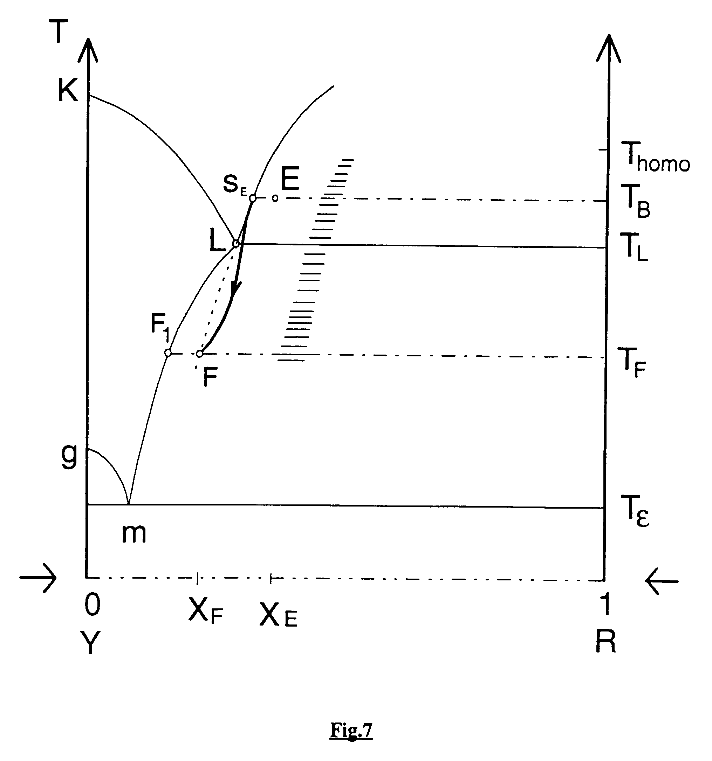

Afin de mieux comparer les procédés classiques et le procédé de l'invention AS3PC, le point initial E est choisi arbitrairement sur les figures 6 et 7, identique au cas précédent; toutefois, comme cela apparaîtra dans les exemples qui suivront, le procédé de l'invention permet de prendre un point E plus écarté du plan A-(RS)-T et donc avec un excès énantiomérique plus important et ainsi d'améliorer la récolte en cristaux de chaque opération.

1 - Cristallisation du premier antipode en excès.

Au début du processus, et contrairement aux protocoles classiques, l'ensemble, cristaux plus solution, n'est plus homogénéisé mais est porté à la température $T_B$. La solution initiale est alors en équilibre avec les cristaux d'énantiomères en excès (par exemple R sur la figure 7). Les points figuratifs de la solution ($S_E$) et de l'ensemble (E) ne sont donc pas confondus dès le début du processus. Ce mélange biphasé est soumis à une loi programmée de descente en température sans ajout de germes cristallins. Le point représentatif de la solution décrit une courbe $S_EF$, contenue dans le plan R-Y-T, dépendante de la cinétique de refroidissement (figure 7). Avec une cinétique correctement ajustée, on obtient au début une croissance des cristaux d'énantiomère en excès, la cristallisation évolue ensuite vers un régime simultané de croissance plus nucléation secondaire. Quand le point représentatif de la solution atteint le point F, on procède à la filtration pour récupérer une masse 2M de cristaux d'antipode R.

2 - Cristallisation du second antipode, cyclicité des opérations.

A partir du point F, qui correspond à la solution-mère précédente, on passe au point E', symétrique de E par rapport au plan vertical A-(RS)-T, par ajout d'une masse 2M de mélange racémique et chauffage à la température $T_B$. L'excès énantiomérique est mis à profit pour se placer dans le domaine biphasé contenant la solution saturée et les cristaux de l'antipode en excès. Au préalable, le mélange racémique ajouté lors du passage de F à E' (comme de F' à E) sera broyé et tamisé afin d'accélérer l'étape de dissolution des deux antipode et plus particulièrement de l'antipode en défaut, et de permettre ainsi la formation d'un nombre important de cristaux de l'antipode en excès jouant le rôle des semences intoduites lors des procédés classiques.

La solution saturée S'$_E$, symétrique de $S_E$ par rapport au plan A-(RS)-T, est soumise à la même loi de refroidissement. Les cristaux présents dès le début du refroidissement croissent et participent ensuite à un double mécanisme de croissance + nucléation secondaire. Comme dans le cas de la première cristallisation, aucun ensemencement n'est donc nécessaire.

Pendant ce temps, le point représentatif de la solution se déplace sur une courbe $S_{E'}F'$ contenue dans le plan de la coupe isoplèthe S-Y'-T symétrique par rapport au plan bisecteur A-(RS)-T.

Au moment où la solution acquiert le point représentatif situé en F', on procède à la filtration pour récolter une masse 2M de cristaux d'antipode S. Une nouvelle addition d'une masse 2M de mélange racémique broyé et tamisé suivie d'une élévation de température à $T_B$ redonne le mélange biphasé à l'équilibre de départ.

La continuation du processus revient alors à répéter ce cycle d'opérations donnant alternativement les cristaux d'antipodes R et S.

3 - <u>Conditions nécessaires pour la mise en oeuvre du procédé AS3PC.</u>

a) Le mélange équimolaire des antipodes optiques réalise, dans le solvant utilisé et pour l'intervalle de température $T_B$ - $T_F$, un conglomérat (antipodes purs ou solvates); toutefois l'existence d'un racémate métastable n'est pas un handicap (cas du tartrate de sodium ammonium tétrahydraté).

b) Les molécules à dédoubler sont stables dans ce solvant et dans la gamme de températures utilisées entre $T_B$ et $T_F$.

c) Les exemples ci-après montrent qu'une détermination des températures d'équilibre ternaire $T_L$ et $T_{HOMO}$ est nécessaire. La température $T_L$ est la température de dissolution du mélange racémique en l'absence de tout excès énantiomérique dans la solution. $T_L$ étant déterminée, la température $T_{HOMO}$ correspond à la température d'homogénéisation de la solution. Elle dépend de l'excès énantiomèrique de départ et du rapport $\alpha$ des solubilités du mélange racémique et de l'antipode à $T_L$. La connaissance des capacités de sursaturation des solutions entre $T_L$ et $T_F$ est également nécessaire, suivant la cinétique de refroidissement, le mode d'agitation, la nature du récipient et la granulométrie des cristaux de l'antipode en excès. En première approximation, le temps d'apparition des cristaux par nucléation primaire dans la solution racémique L homogène, refroidie à partir d'une température légèrement supérieure à $T_L$ avec la même cinétique, donne une indication sur la capacité de sursaturation tolérée par le conglomérat dans ces conditions expérimentales. Cette façon d'opérer a été prise en compte dans les exemples.

d) La connaissance de la cinétique de dissolution d'une masse connue de mélange racémique (de granulométrie donnée) dispersée dans la solution à la température $T_B$. Quelques essais suffisent pour connaître cette durée.

4 - <u>Intérêts du procédé de l'invention AS3PC par rapport aux procédés SIPC, S3PC ou leurs variantes.</u>

Les exemples ci-après montrent que le procédé AS3PC est bien adapté aux différentes classes d'énantiomères susceptibles d'être dédoublés : composés covalents ou à caractère ionique, solvatés ou non.

Les résultats obtenus illustrent les avantages de ce procédé par rapport aux procédés SIPC, S3PC et à toutes les variantes, en particulier :

- levée de la contrainte de l'ensemencement,
- amélioration du rendement et de la pureté optique,
- meilleure reproductibilité de chaque cristallisation,
- diminution de la durée de chaque cycle.

Une discussion systématique de ces avantages est détaillée ci-dessous.

a) <u>Levée de la contrainte de l'ensemencement.</u>

- Les germes nécessaires à chaque cristallisation sont ici constitués par les cristaux en équilibre dans le domaine biphasé : solution saturée + cristaux de l'antipode en excès. Cette phase cristalline possède donc, a priori, une pureté optique maximale. Dans le cas de solution solide limitée, l'atteinte de l'équilibre permet d'avoir la pureté optique maximale des germes dans la solution à cette température.
- Le procédé AS3PC ne nécessitant pas d'introduction de germes, moins de poussières ou d'impuretés solides sont introduites dans le milieu (il n'y aura en fait que celles introduites avec le mélange racémique broyé et tamisé).
- Les cristaux formés in situ sont d'emblée bien dispersés dans la solution soumise à une agitation.
- Lors de la mise en oeuvre du procédé AS3PC, seul un mélange partiellement enrichi est nécessaire; par exemple la forme chirale naturelle (cas du tartrate) ou celle obtenue par diastéréoisomère avec un agent de dédoublement naturel, tel qu'un alcaloïde naturel. Dès la deuxième manipulation et pour toutes les suivantes, l'absence d'introduction de germes rend caduque la nécessité d'isoler et purifier le second antipode.

Le principe de l'auto-ensemencement selon le procédé de l'invention AS3PC peut être étendu à la séparation de sels diastéréoisomères, énantiomères l'un de l'autre et réalisant, pour l'intervalle de température défini, la paire stable dans le système quaternaire réciproque des deux paires de sels diastéréoisomères en présence du solvant.

b) <u>Amélioration de la pureté et du rendement.</u>

- La température $T_B$, choisie en première approximation comme étant égale à la moyenne des valeurs $T_{HOMO}$ et $T_L$, on peut (en supposant une variation linéaire de la solubilité dans cet étroit intervalle de température) considérer que la masse de germes à l'état initial représente le quart de la masse de cristaux obtenue à chaque récolte. Dans la plupart des cas, on peut, en ajustant $T_B$ plus près de $T_L$, avoir jusqu'à 40% de la masse de la récolte finale présente sous forme de fins cristaux. Ceci est aisé à réaliser quand la variation de $T_{HOMO}$ en fonction de l'excès énantiomérique présente une pente suffisante et que l'excès énantiomérique issu de chaque filtration est important (comme cela est montré dans certains des exemples ci-après). En tout état de cause 50 % représente la limite que l'on peut atteindre à l'équilibre; dans ce cas la température $T_B$ serait égale à la température $T_L$. Cette masse importante de fins cristaux permet, quand la température diminue, d'obtenir une large surface équivalente de cristaux et donc de privilégier le processus de croissance plutôt que d'induire directement une nucléation à forte sursaturation comme dans le procédé SIPC et dans une moindre mesure, dans les procédés regroupés sous le sigle "S3PC".

- La loi de programmation de descente en température permet de maîtriser le processus de croissance d'une part, et de nucléation + croissance d'autre part, afin de l'adapter à chaque cas d'espèce. Une cinétique lente de refroidissement permettra au point représentatif du point-solution de se déplacer le plus près possible de la courbe de solubilité à caractère stable puis métastable de l'antipode (Mullin, J. W. (1972) Ed. Butterworth) . Dans le cas où la nucléation secondaire de l'antipode en excès est difficile (cas du dédoublement du ($\pm$) chlorhydrate de l'acide glutamique) (Noguchi Institute (1968) brevet du Royaume-Uni n° 1 197 809), cet avantage est déterminant.

- Pour un même point ensemble de départ, le procédé AS3PC permet ainsi, au point figuratif de la solution, de rester plus éloigné de la zone dite labile de métastabilité d'Ostwald (zone hachurée de nucléation spontanée des énantiomères et/ou de racémate métastable éventuel, des figures 5 et 7) que le procédé SIPC et ses variantes. Le procédé AS3PC sera donc le mieux adapté dans les cas difficiles à mettre en oeuvre où apparaissent des contraintes supplémentaires comme :

  .   les capacités de sursaturation des solutions quasi-racémique sont faibles,
  .   le rapport $\alpha$ est défavorable,
  .   l'existence d'un racémate métastable mais possédant une forte vitesse de nucléation primaire dans la gamme de température utilisée.

Cela signifie encore que pour un couple d'énantiomères donné, c'est à dire une même capacité de sursaturation et un même rapport de solubilité $\alpha$, le procédé AS3PC permet d'obtenir un meilleur rendement. La cristallisation de chaque antipode pourra être poursuivie plus longtemps sans que le point solution atteigne la composition définie par la limite d'Ostwald. Le procédé AS3PC permet donc d'affecter aux mélanges E, F, E', F', de début et de fin de cristallisation, un excès énantiomérique plus important que dans le procédé SIPC ou ses variantes. Une cristallisation plus lente permet également d'obtenir des solides présentant moins de défauts de structure et notamment des inclusions de solutions-mères, dans les cristaux. Tous les exemples montrent ainsi une amélioration de la pureté optique des cristaux.

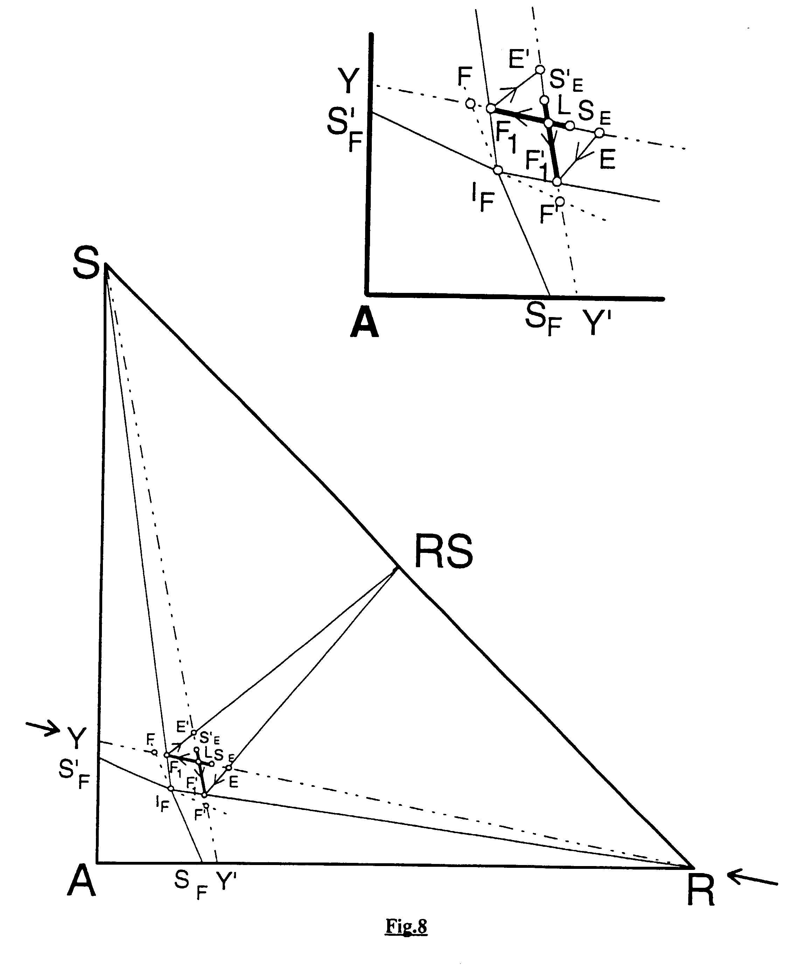

Pour un rapport $\alpha$ tel que $S(\pm) < 2\text{-}\alpha$ ($\alpha$ = rapport des solubilités du mélange racémique et de l'antipode à $T_F$: $\alpha = S(\pm) / S_{(R)}$), le point $F_1$ peut se substituer au point F pour la fin de filtration. D'une manière générale, pour une coupe isoplèthe donnée, c'est de $F_1$ et F le point de plus faible excès énantiomérique qui pratiquement représentera la composition finale des liqueurs-mères soumises à la filtration (figure 8).

c) <u>Meilleure reproductibilité.</u>

- Les paramètres : temps de maintien à la température $T_B$, valeur de l'excès énantiomérique, granulométrie des cristaux à $T_B$, vitesse et mode d'agitation, loi de programmation de descente en température étant définis, le procédé AS3PC montre une bonne reproductibilité; la taille des cristaux peut être ajustée et l'on peut notamment moduler les paramètres afin d'éviter l'existence de cristaux de taille réduite (fines) gênant la filtration.
- L'utilisation d'une tempéraure maximale plus basse pendant un temps limité, $T_B < T_D$, implique que le procédé AS3PC évitera, mieux que le procédé SIPC ou ses variantes, la dégradation et la racémisation des antipodes dans le cas d'une fragilité des molécules à dédoubler dans le solvant utilisé. Cette propriété concerne également le solvant et elle peut se révéler importante dans la mesure où les solutions-mères sont recyclées en permanence.

d) <u>Durée de chaque cycle.</u>

- L'étape d'homogénéisation de la solution-mère (à $T > T_{HOMO}$) étant inexistante, le procédé AS3PC contribue directement à un gain d'énergie et à des durées de chaque cycle plus courtes, car la croissance s'établit à partir d'une masse importante de cristaux et ceci dès le début du refroidissement. Ce gain est appréciable si le mélange racémique introduit après chaque filtration est finement broyé et tamisé (de préférence peu de temps avant l'addition).
- L'étape de dissolution du mélange racémique (en fait de l'antipode en défaut plus que de l'antipode en excès dans la solution issue de la filtration), peut être encore accélérée par un chauffage pendant une durée limitée à une température $T > T_B$, suivi d'un retour rapide à $T_B$. La durée est ajustée afin que tout l'antipode en excès soit dissous en un temps minimun. Cette durée dépend de la cinétique de dissolution avec, pour paramètres :

.   la masse et la granulométrie du mélange racémique,
.   la loi de programmation de la température,
.   le type et la vitesse d'agitation,

tous les autres paramètres étant fixés.

Cette opération a pour effet (comme d'ailleurs l'attente de l'établissement de l'équilibre à $T_B$, mais pour cette dernière de façon moins prononcée) de dissoudre les cristaux les plus fins, ce qui présente l'inconvénient de diminuer la surface disponible pour la croissance. Cet effet sera fortement limité par un broyage et un tamissage du mélange racémique ajouté à chaque étape, et une vitesse d'agitation importante. Si nécessaire, on utilise les ultra-sons (en tenant compte de leur effet thermique) afin d'obtenir un spectre granulométrique plus fin, plus homogène et reproductible. On peut également utiliser un mélange racémique obtenu par lyophilisation partielle afin d'accélérer l'étape de dissolution et disposer d'un spectre de granulométrie fin des cristaux de départ.

La maîtrise des paramètres améliore la reproductibilité de la cristallisation et facilite donc une étude d'automatisation du procédé.

V - <u>EXEMPLES</u>.

1 - <u>Dispositif expérimental.</u>

Les opérations sont effectuées alternativement dans deux tubes d'environ 12 cm de hauteur et de 29 mm de diamètre à col rodé (29/32 n°4). Ces tubes sont munis, dans leur partie supérieure, d'un tube latéral pour établir une dépression nécessaire lors de la filtration. Les cristaux sont récupérés sur verre fritté n° 2 ou 3, adaptable sur chaque tube par l'intermédiaire d'un anneau de caoutchouc. L'agitation est assurée par un barreau magnétique. Les liqueurs passent successivement d'un tube à l'autre. Ces transferts, réduits au maximun, n'empêchent pas des pertes entre chaque opération. Plus les quantités de produit utilisé seront faibles, plus les pertes seront proportionnellement grandes. On peut les répertorier en deux catégories :

- Pertes, au niveau du verre fritté et dans le tube initial, de liqueur-mère contenant l'excès énantiomérique de fin de cristallisation. La compensation se fait par ajout de cristaux racémiques et de solvant, de telle sorte que cette addition corresponde au mélange L, comme indiqué dans les tableaux de résultats au niveau de la colonne "compensation".
- Pertes en solvant principalement dues à la filtration créée par dépression. La compensation se fait par ajout à chaque opération de solvant supplémentaire.

Dans certains cas, par exemple lorsque l'on utilise un solvant très volatil, le procédé de compensation doit être plus précis. Une petite quantité de la solution est prélevée, afin d'en déterminer la composition, permettant ensuite une compensation rigoureuse.

Afin d'accéder à une bonne reproductibilité des résultats, le liquide caloporteur circulant dans la double enveloppe de la chambre de cristallisation est régulé en température avec une précision de $+ 0,1°$ C. L'appareillage mis en oeuvre permet de fixer une loi de refroidissement reproductible.

Les exemples ci-après traités par cristallisation discontinue (ou en batch), pourraient l'être, sur le même principe, de façon continue ou semi-continue.

2 - <u>Dédoublement du 3-5-dinitrobenzoate-1-pényléthanol.</u>

Cet ester, dérivé cristallisé du 1-phényéthanol (Synthon chiral courant), a fait l'objet d'une séparation par la méthode classique (Brienne, M. J., Collet, A. et Jacques, J. (1983) Synthesis 9, 704-5). Le dédoublement de ce dérivé covalent a été repris en comparant les méthodes SIPC et AS3PC.

a) <u>Caractéristiques du produit.</u>

- Température de fusion de l'antipode : 123°C.
- Température de fusion du mélange racémique : 95 °C.
- Pouvoir rotatoire spécifique à 20 °C, c = 1g/100ml, toluène.

| λ (nm) | 436 | 589 |
|---|---|---|
| $[\alpha]^{20}$ (°) | 93,9 | 39,0 |

b) Dédoublement par la méthode AS3PC.

- Conditions liées aux équilibres.

  . Solubilité dans le toluène des mélanges racémiques :

| T (°C) | 20 | 27,9 |
|---|---|---|
| Solubilité (% massique) | 27,4 | 34,6 |

  . Solubilité de l'antipode pur (-) : 13,2 % à 20 °C; rapport $\alpha$ = 2,07,
  . Coordonnées du point L : Concentration = 34,6 % massique; température = 27,9 °C.
  . Evolution de $T_{HOMO}$ avec l'excès énantiomérique : (mélange racémique / (solvant + mélange racémique)) = 34,6 % = constante.

| % Antipode (-) | 0 | 3 | 5 | 7 | 9 |
|---|---|---|---|---|---|
| $T_{HOMO}$ (°C) | 27,9 | 30 | 31,2 | 32,3 | 33,6 |

- Conditions liées à la cinétique.

  . Température $T_B$ : 29,5 °C.
  . Température $T_F$ : 19,5 °C.
  . Loi de refroidissement : T = f(t) :

| T (°) | 29,5 | 28,1 | 25,1 | 22,1 | 19,5 |
|---|---|---|---|---|---|
| t (mn) | 0 | 10 | 20 | 30 | 40 |

  . Durée de l'état de sursaturation de la solution L soumise à cette loi de refroidissement : environ 60 minutes pour une vitesse d'agitation de 250 tours/mn.
  . Durée de cristallisation : 42 minutes.

- Conditions initiales.

  . Excès énantiomérique initial : 9 % .

| masse solvant (g) | masse (±) (g) | masse (-) (g) |
|---|---|---|
| 25,05 | 13,25 | 1,31 |

  . Durée du palier à $T_B$ : 60 minutes.
  . Vitesse d'agitation : 125 tours/mn à 29,5 °C; 225 tours /mn à 19,5 °C.

- Resultats.
  Grâce à une taille importante des cristaux, la filtration, effectuée sur fritté n°2, est aisée.

| N° | masse antipode pur (g) | % pureté optique | Compensation sol. (±) 34,6 % |
|---|---|---|---|
| 1 | 2,46 | (-) 88,8 | 1,86 |
| 2 | 2,40 | (±) 94,3 | 1,01 |
| 3 | 2,36 | (-) 94,4 | 1,86 |

(suite)

| N° | masse antipode pur (g) | % pureté optique | Compensation sol. (±) 34,6 % |
|---|---|---|---|
| 4 | 2,55 | (±) 85,9 | 1,96 |
| 5 | 2,48 | (-) 87,0 | 1,26 |
| 6 | 2,38 | (±) 94,3 | 1,46 |
| 7 | 2,61 | (-) 90,6 | 1,50 |
| 8 | 2,68 | (±) 94,2 | 1,09 |
| 9 | 2,36 | (-) 88,6 | 1,66 |
| 10 | 2,28 | (±) 92,0 | ---- |

Après chaque entraînement, en plus de la compensation en solution racémique, indiquée dans le tableau ci-dessus, il a été ajouté en moyenne, 0,7 g de toluène.

- Masse moyenne de critaux d'antipode pur : 2,55 g.
- Excès énantiomérique moyen : 1,23 g soit 8,5% soit encore 9% sans perte de solution-mère.
- Pureté optique moyenne : 91 %.

c) Dédoublement par la méthode SIPC.

- Conditions initiales.
  Le premier entraînement par la méthode SIPC est effectué sur une solution présentant un excès énantiomérique de 8,87%.

- Température $T_D$: 35 °C.
- Température $T_F$: 20 °C.
- Temps à $T_F$ avant l'introduction des germes : 5 mn.
- Masse de germes : 20 mg.
- Temps de cristallisation : 15 minutes.

- Résultats.
  La filtration sur fritté N°2, est moins aisée que par le procédé de l'invention, en raison de la finesse des cristaux.
  Au-delà de 10 minutes de cristallisation, l'ensemble solution plus cristaux, prend l'apparence de gel, l'agitation n'est plus homogène sur toute la hauteur du tube, malgré une augmentation de la vitesse d'agitation.

| N° | masse antipode % pur (g) | % pureté optique | Compensation sol. (±) 34,6 % |
|---|---|---|---|
| 1 | 1,76 | (-) 75,8 | 2,11 |
| 2 | 1,28 | (+) 64,1 | 1,99 |
| 3 | 1,63 | (-) 69,9 | 2,54 |
| 4 | 1,65 | (±) 74,0 | ---- |

- Masse moyenne d'antipode pur : 1,58 g.
- Excès énantiomérique moyen : 0,79 g soit 5,63 %.
- Pureté optique moyenne : 70,9 %.

d) Résultats obtenus par Brienne, J. M. et al. (Synthesis 9 (1983) 704-5).

- Conditions initales.

| masse solvant (g) | masse (±) (g) | masse (-) (g) |
|---|---|---|
| 125,4 | 66,2 | 4,4 |

- Concentration en mélange racémique : 34,6 %.
- Excès énantiomérique initial : 6 %.
- Température $T_D$ : 60 °C.
- Température $T_F$ : 20 °C.
- Masse de germes : 100 mg.
- Temps de cristallisation : 60 mn.
- Vitesse d'agitation : 150 tours/mn.

- Résultats.

- Masse moyenne d'antipode pur : 8,3 g soit, en ramenant à la même masse de solution racémique que celle utilisée pour les essais, 1,66g.
- Excès énantiomérique moyen : 4,15 g, soit 5,89 %.
- Pureté optique moyenne : 83 %.

Les résultats obtenus avec la méthode SIPC sont moins bons que ceux observés avec le même procédé SIPC par Brienne et al. (Synthesis 9 (1983) 704-5) pour les raisons suivantes :

- La méthode a été mise en oeuvre avec des masses cinq fois plus faibles, ce qui implique un rapport surface / volume moins favorable.
- Les pertes dues à la filtration restent équivalentes en masse quelle que soit la masse totale mise en jeu, elles sont donc proportionnellement plus élevées dans ce cas.
- L'agitation utilisée par Brienne, M. J. et al. est une hélice, ne provoquant donc pas de frottements sur les parois du récipient de verre. En effet, les frottements induisent une forte nucléation, c'est pourquoi le temps de cristallisation dans le cas du présent exemple (15 mn), pour une solution initiale identique, est nettement inférieur aux 60 mn indiqués par les auteurs.

Il convient de noter que ces conditions défavorables sont également présentes lors de la mise en oeuvre de la méthode AS3PC. Les résultats nettement meilleurs obtenus avec la méthode de l'invention montrent qu'elle offre, par rapport aux résultats obtenus avec la méthode SIPC dans des conditions plus favorables, des avantages significatifs qui compensent très largement ces handicaps.

3 - Dédoublement du tartrate double de sodium et d'ammonium tétrahydraté.

a) Caractéristique du produit.

Il s'agit du sel de Pasteur dont les équilibres du système ternaire R-S-$H_2O$ montrent l'existence d'un racémate stable de formule (±)$NaNH_4(CHOHCOO)_2$, $2H_2O$ (sel de Sacchi), dès 27 °C. Ce composé intermédiaire est à caractère métastable dans la gamme de température comprise entre 12 et 18 °C.

- Pouvoir rotatoire spécifique, 20 °C, c = 1g/100 ml, eau.

| $\lambda$ (nm) | 365 | 589 |
|---|---|---|
| $[\alpha]_{20}$ (°) ) | +60,4 | +23,3 |

b) Dédoublement par la méthode AS3PC.

- Conditions liés aux équilibres.

- Solubilités dans l'eau des mélanges racémiques :

| T (°C) | 12 | 16,0 | 16,3 | 17,3 |
|---|---|---|---|---|

(suite)

| Solubilité (% massique) | 42,5 | 45,0 | 46,0 | 46,5 |
|---|---|---|---|---|

. Solubilité de l'antipode R : 32 % à 12 °C; rapport $\alpha$ = 1,33 à 12 °C.
. Coordonnées du point L : 46% massique, température 16,3 °C.
. Evolution de $T_{HOMO}$ avec l'excès énantiomérique : (mélange racémique / (solvant + mélange racémique)) = 46% = constante.

| % antipode | 0 | 3 | 5 |
|---|---|---|---|
| $T_{HOMO}$ (°C) | 16,3 | 17,4 | 18,3 |

- <u>Conditions liées à la cinétique.</u>

. Température $T_B$ : 17,3 °C.
. Température $T_F$ : 12 °C.
. Loi de refroidissement : T = f(t) :

| T (°C) | 17,3 | 17,0 | 16,5 | 15,4 | 14,0 | 13,0 | 12,1 | 12,0 | 12,0 |
|---|---|---|---|---|---|---|---|---|---|
| temps (mn) | 0 | 3 | 5 | 8 | 12,5 | 16 | 20 | 25 | 30 |

. La durée de l'état de sursaturation de la solution homogène L soumise à cette cinétique est supérieure à 40 minutes pour une vitesse d'agitation de 150 tours/mn.
. Durée de la cristallisation : 20 mn, sauf dans le cas des opérations 14 à 16 pour lesquelles la durée est de 21,5 mn.

- <u>Conditions initiales.</u>

. Excès énantiomérique initial : 4,5 %.

| masse solvant (g) | masse (±) (g) | masse (+) (g) |
|---|---|---|
| 5,67 | 4,83 | 0,23 |

. Durée du palier à la température $T_B$ : 40 mn pour un mélange racémique broyé sur tamis de 250 µ quelques minutes avant son introduction dans le tube de cristallisation.
. Vitesse d'agitation : 150 tours/mn.

- <u>Résultats.</u>

| N° | masse antipode pur (g) | % pureté optique | Compensation sol. (±) 46% |
|---|---|---|---|
| 1 | 0,47 | (+) 94,7 | 0,50 |
| 2 | 0,52 | (-) 94,4 | 0,85 |
| 3 | 0,53 | (+) 96,1 | 0,70 |
| 4 | 0,56 | (-) 92,5 | 0,88 |
| 5 | 0,55 | (+) 95,5 | 0,64 |
| 6 | 0,56 | (-) 91,1 | 0,66 (i) |
| 7 | 0,59 | (+) 97,2 | 0,62 |
| 8 | 0,59 | (-) 97,5 | 0,79 |
| 9 | 0,52 | (+) 97,1 | 0,60 |

(suite)

| N° | masse antipode pur (g) | % pureté optique | Compensation sol. (±) 46% |
|----|------------------------|------------------|---------------------------|
| 10 | 0,53 | (-) 96,0 | 0,72 |
| 11 | 0,56 | (+) 96,1 | 0,64 |
| 12 | 0,53 | (-) 94,4 | 0,58 |
| 13 | 0,52 | (+) 96,0 | 0,70 (i) |
| 14 | 0,61 | (-) 97,0 | 0,64 |
| 15 | 0,62 | (+) 96,7 | 0,56 |
| 16 | 0,61 | (-) 95,4 | ---- |

Les cristaux obtenus à chaque fin de processus sont facilement filtrables sur fritté n° 2; ils retiennent fort peu de solution-mère, comme le montrent les puretés optiques, malgré la concentration élevée des liqueurs.

En plus des pertes habituelles, on observe, pour ce composé des pertes en ammoniac dues également à la filtration par dépression. La compensation est effectuée par ajout de 40 mg environ d'une solution d'ammoniaque concentrée qui conduit à un léger excès en cation ammonium; celle-ci est notée (i) dans la colonne compensation du tableau ci-dessus. Cet excès d'ammoniaque améliore légèrement la pureté des cristaux récupérés. En cas de défaut d'ammoniaque, un trouble très fin, persistant même à 20°C, se manifeste dans les liqueurs.

- . Masse moyenne de cristaux d'antipode pur (manipulations 2 à 13) : 0,55 g.
- . Excès énantiomérique moyen : 0,27 g soit 5,3 %.
- . Pureté optique moyenne : 95,5%.

Malgré l'existence d'un racémate métastable et les faibles quantités de solution racémique mises en jeu (10,5 g), la méthode AS3PC donne donc de bons résultats avec cet hydrate .

b) Dédoublement par la méthode SIPC.

- Conditions initiales.

| masse solvant (g) | masse (±) (g) | masse (+) (g) |
|-------------------|---------------|---------------|
| 5,67 | 4,83 | 0,23 |

- . Concentration de la solution racémique : 46 %.
- . Excès énantiomérique initial : 4,5 %
- . Température $T_D$ : 18,6 °C.
- . Température $T_F$ : 12 °C.
- . Temps à $T_F$ avant l'introduction des germes : 10 à 12 mn.
- . Masse de germes : 5 mg.
- . Temps de cristallisation : 10 mn.

- Résultats.
  Filtration sur fritté n°2.

| N° | masse antipode pur (g) | % pureté optique | Compensation sol. (±) 46% |
|----|------------------------|------------------|---------------------------|
| 1 | 0,38 | (+) 92 | 0,51 |
| 2 | 0,42 | (-) 88 | 0,55 |
| 3 | 0,39 | (+) 92 | 0,57 |
| 4 | 0,39 | (-) 94 | ---- |

- . Masse moyenne d'antipode pur : 0,40 g.

. Excès énantiomérique moyen : 0,20 g soit 4%.
. Pureté optique moyenne : 91,5 %.

La méthode AS3PC fournit de meilleurs résultats notamment pour les masses en cristaux d'antipode pur.

4 - Dédoublement du chlorhydrate de l'acide glutamique.

Le brevet anglais n° 1 197 809 au nom de Noguchi Institute décrit le dédoublement de ce sel de l'acide glutamique en utilisant un procédé polythermique avec ensemencement de la solution homogène (S3PC).

a) Caractéristiques du produit.

| $\lambda$ (nm) | 365 | 589 |
|---|---|---|
| $[\alpha]20(°)$ $H_2O$ | 74,7 | 20,6 |
| $[\alpha]20(°)$ HCl, 1N | 94,5 | 26,3 |

b) Dédoublement par la méthode AS3PC.

- Conditions liées aux équilibres.

. Solubilité du mélange racémique dans l'eau :

| Température (°C) | 30 | 42,8 |
|---|---|---|
| Solubilité (% massique) | 37,5 | 49 |

. Coordonnées du point L : 49% massique; température : 42,8 °C.
. Evolution de $T_{HOMO}$ avec l'excès énantiomérique : (mélange racémique / (solvant mélange racémique)) = 49% = constante.

| % antipode (+) | 0 | 3 | 4,5 | 6 | 9 |
|---|---|---|---|---|---|
| $T_{HOMO}$ (°C) | 42,8 | 45,0 | 46,8 | 48,1 | 51,1 |

- Conditions liées à la cinétique.

. Température $T_B$ : 44,4°C.
. Température $T_F$ : 30°C,
. Cinétique de refroidissement : T = f(t) :

| t (mn) | 0 | 15 | 45 | 55 |
|---|---|---|---|---|
| T (°C) | 44,4 | 37,0 | 32,0 | 30,0 |

. Durée de l'état de sursaturation de la solution L soumise à cette cinétique : 120 mn pour une vitesse d'agitation de 150 tours/ mn.
. Durée de cristallisation : 55 mn.

- Conditions initiales.

. Excès énantiomérique initial : 9,24 %.

| masse solvant (g) | masse ($\pm$) (g) | masse (+) (g) |
|---|---|---|
| 20,50 | 19,50 | 1,99 |

. Durée du palier à $T_B$ : 30 mn.

. Vitesse d'agitation : 150 tours/mn au début de la cristallisation, puis 250 tours/mn à la fin.

- Résultats.

Filtration sur fritté n°3.

| N° | masse antipode pur (g) | % pureté optique | Compensation sol. (±) 49% |
|---|---|---|---|
| 1 | 3,88 | (+) 93,6 | 2,26 |
| 2 | 3,97 | (-) 95,6 | 2,86 |
| 3 | 4,40 | (+) 94,8 | 2,01 |
| 4 | 4,27 | (-) 94,9 | 1,36 |
| 5 | 4,43 | (+) 94,9 | 1,77 |
| 6 | 4,52 | (-) 94,5 | 1,34 |
| 7 | 4,73 | (+) 96,0 | 1,60 |
| 8 | 4,53 | (-) 91,8 | 1,30 |
| 9 | 4,43 | (+) 97,1 | 1,35 |
| 10 | 4,72 | (-) 96,2 | ---- |

. Masse moyenne d'antipode pur : 4,50 g à partir de la manipulation N°3.
. Excès énantiomérique moyen : 2,25 g soit 10,34%.
. Pureté optique moyenne : 95%.

c) Résultats obtenus par la méthode S3PC décrite dans le brevet anglais n° 1 197 809, par la méthode S3PC.

- Conditions initiales.

| masse solvant (g) | masse (±) (g) | masse (+) (g) |
|---|---|---|
| 26,10 | 25,18 | 2,75 |

. Concentration du mélange racémique 51,7 % massique.
. Excès énantiomérique initial : 9,8 %.
. Température $T_D$ : 56 °C (solution homogène).
. Température TF : 32 °C.
. Masse de germes introduits : 1 g.
. Vitesse de refroidissement: 5 à 8 °C /heure.
. Durée de cristallisation : 150 mn.

- Resultats.

. Masse d'antipode pur : 5,73 moins 1,00 g de germes égal 4,73 g, soit, en ramenant à la même masse de solution racémique que celle utilisée pour les essais : 3,70 g.
. Excès énantiomérique : 2,37 g soit 8,6 %.
. Pureté optique : 72,6 %.

La masse d'antipode obtenue et la pureté optique sont supérieures par la méthode AS3PC.

5 - Dédoublement de la thréonine.

Cet exemple permet de comparer les résultats obtenus par la méthode AS3PC avec les résultats des travaux de Amiard, G. (Bull. Soc. Chim. (1956) 447) effectués par cristallisation préférentielle avec nucléation spontannée.

a) Caractéristiques du produit

. Pouvoir rotatoire spécifique, 20°C, c = 1 g/100ml, eau :

| λ (nm) | 365 | 589 |
|---|---|---|
| [α]20(°) | - 81,0 | - 28,1 |

b) Dédoublement par la méthode AS3PC.

- Conditions liées aux équilibres.

. Solubilités dans l'eau des mélanges racémiques :

| T °C | 30,0 | 54,5 |
|---|---|---|
| solubilité (% massique) | 14,5 | 23,1 |

. Solubilité de l'antipode : 9,1 % à 30 °C, rapport α = 1,59.
. Coordonnées du point L : 23,08 % massique, température $T_L$: 54,5 °C.
. Evolution de $T_{HOMO}$ avec l'excès énantiomérique (mélange racémique / (solvant + mélange racémique)) = 23,08 % = constante.

| % antipode (-) | 0 | 2 | 4 | 6 | 8 |
|---|---|---|---|---|---|
| $T_{HOMO}$ (°C) | 54,5 | 56 | 61,5 | 65 | 68,5 |

- Conditions liées à la cinétique.

. Température $T_B$ : 58 °C.
. Température $T_F$ : 31°C.
. Cinétique de refroidissement : T = f(t) :

| T (°C) | 58 | 55 | 51 | 47 | 43 | 39 | 34 | 31 |
|---|---|---|---|---|---|---|---|---|
| t (mn) | 0 | 12 | 22 | 30 | 38 | 46 | 54 | 60 |

. Durée de l'état de sursaturation de la solution L soumise à cette cinétique : 80 mn.
. Durée de cristallisation : 60 mn.

- Conditions initiales.

. Excès énantiomérique initial : 5%.

| masse solvant (g) | masse (±) (g) | masse (+) (g) |
|---|---|---|
| 30,00 | 9,00 | 0,47 |

- Résultats.

| N° | masse antipode pur (g) | % pureté optique | Compensation sol. (±) 23,1 % |
|---|---|---|---|
| 1 | 1,02 | (+) 97,5 | 1,432 |
| 2 | 1,20 | (-) 94,0 | 1,769 |
| 3 | 1,46 | (+) 93,0 | 1,663 |
| 4 | 1,65 | (-) 97,5 | 1,443 |
| 5 | 1,60 | (+) 98,3 | 0,552 |

(suite)

| N° | masse antipode pur (g) | % pureté optique | Compensation sol. (±) 23,1 % |
|---|---|---|---|
| 6 | 1,64 | (-) 98,5 | 1,687 |
| 7 | 1,72 | (+) 99,1 | 1,650 |
| 8 | 1,70 | (-) 92,4 | 1,800 |
| 9 | 1,66 | (+) 91,8 | 1,842 |
| 10 | 1,69 | (-) 97,8 | ---- |

. Masse moyenne de cristaux d'antipode pur (à partir de la manipulation N° 4) : 1,665 g.
. Excès énantiomérique moyen 0,833 g, soit 8,47 %.
. Pureté optique moyenne : 96,5 %.

c) Résultats obtenus par Amiard.

- Conditions initiales.

| masse solvant (g) | masse (±) (g) | masse (+) (g) |
|---|---|---|
| 150 | 45 | 5 |

. Concentration du mélange racémique : 23,08%.
. Excès énantiomérique : 10 %.
. Température $T_D$ : 80 °C.
. Température $T_F$ : 20 °C.
. Masse de germes : nucléation spontanée à 30°C sans agitation, après une durée non précisée par l'auteur.
. Temps de cristallisation : 60 mn (en agitant de temps en temps).

- Résultats.

. Masse moyenne d'antipode pur : 7,80 g soit, en ramenant à la masse de solution racémique utilisée dans les essais : 1,55 g.
. Excès énantiomérique moyen : 3,9 g soit 8%.
. Pureté optique moyenne : 84,2 %.

L'utilisation de la méthode AS3PC a permis l'obtention d'une récolte d'antipode supérieure avec beaucoup plus de régularité et surtout avec une pureté optique nettement améliorée.

6 - Dédoublement de la 5-méthyl-5-phénylhydantoïne.

a) Caractéristiques du produit.
Température de fusion de l'antipode 242 °C.
Température de fusion du mélange racémique : 196°C.
Pouvoir rotatoire spécifique à 20°C, c = 1g/100ml, éthanol.

| λ(nm) | 589 | 578 | 546 | 436 | 365 |
|---|---|---|---|---|---|
| [α]20(°) | 116 | 122 | 139 | 229 | 433 |

b) Dédoublement par la méthode AS3PC.

- Conditions liées aux équilibres.

. Solubilité des mélanges racémiques dans le 2-méthoxy-éthanol :

| T (°c) | 20,2 | 30,2 | 34,8 | 38,0 | 41,9 |
|---|---|---|---|---|---|
| Solubilité s (±) (% massique) | 18,1 | 20,0 | 21,2 | 21,8 | 22,9 |
| Solubilité s(+) (% massique) | 9,8 | 10,9 | 11,5 | | |
| Rapport $\alpha$ | 1,84 | 1,84 | 1,84 | | |

. Coordonnées du point L : 21,48 % massique, température $T_L$ : 37 °C.
. Evolution de $T_{HOMO}$ avec l'excès énantiomérique (mélange racémique / (solvant + mélange racémique)) = 21,48 % = constante :

| % énantiomère | 0 | 2 | 4 | 6 |
|---|---|---|---|---|
| $T_{HOMO}$ (°C) | 37,0 | 39,8 | 42,7 | 45,5 |

- Conditions liées à la cinétique.

. Température $T_B$ : 40 °C.
. Température $T_F$: 20 °C.
. Cinétique de refoidissement : T = f(t) :

| T (°C) | 40 | 35 | 30 | 25 | 20 | 20 |
|---|---|---|---|---|---|---|
| t (mn) | 0 | 12 | 22 | 30 | 38 | 46 |

. Durée de l'état de sursaturation de la solution L soumise à cette cinétique : supérieur à 90 minutes pour une vitesse d'agitation de 150 tours/mn.
. Durée de cristallisation : 60 minutes.

- Conditions initiales.

. Excès énantiomérique initial : 5 %.

| masse solvant (g) | masse (±) (g) | masse (+) (g) |
|---|---|---|
| 15,704 | 4,296 | 0,229 |

. Durée du palier à $T_B$ : 30 minutes
. Vitesse d'agitation : 100 tours/mn au début de la cristallisation, puis 150 tours à la fin.

- Résultats.

| N° | masse antipode pur (g) | % pureté optique | Compensation sol. (±) 23,1 % |
|---|---|---|---|
| 1 | 0,560 | (+) 94 | 2,224 |
| 2 | 0,551 | (-) 89 | 1,632 |
| 3 | 0,629 | (+) 92 | 1,174 |
| 4 | 0,561 | (-) 92 | 1,095 |
| 5 | 0,599 | (+) 91 | 0,967 |
| 6 | 0,626 | (-) 92 | 1,034 |
| 7 | 0,510 | (+) 91 | 0,942 |
| 8 | 0,521 | (-) 87 | 1,181 |
| 9 | 0,568 | (+) 89 | |

- Masse moyenne de cristaux d'antipode pur : 0,569 g.
- Excès énantiomérique moyen 0,285 g, soit 6,2 %.
- Pureté optique moyenne : 91 %.

7 - Dédoublement de la 5-méthyl,5-(4 méthyl phényl)-hydantoïne.

a) Caractéristiques du produit.

- Température de fusion de l'antipode : 250 °C.
- Température de fusion du mélange racémique : 205°C.
- Pouvoir rotatoire spécifique à 20°C, c = 1g/100ml, éthanol.

| $\lambda$(nm) | 589 | 578 | 546 | 436 | 365 |
|---|---|---|---|---|---|
| $[\alpha]20(°)$ | 105 | 110 | 127 | 234 | 418 |

b) Dédoublement par la méthode AS3PC.

- Conditions liées aux équilibres.

  . Solubilité des mélanges racémiques dans le 2-méthoxy-éthanol :

| T(°C) | 15 | 25 | 39 | 45 | 50 |
|---|---|---|---|---|---|
| Solubilité s (% massique) | 12,9 | 14,4 | 17,0 | 19,5 | 20,1 |

  . Solubilité de l'antipode R : 7,5 % à 25°C, rapport $\alpha = 1,92$ à 25 °C.
  . Coordonnées du point L : 17 % massique, température $T_L$ : 39 °C.
  . Evolution de $T_{HOMO}$ avec l'excès énantiomérique (mélange racémique / (solvant + mélange racémique)) = 17 % = constante :

| % énantiomère | 0 | 2 | 4 | 6 | 8 |
|---|---|---|---|---|---|
| $T_{HOMO}$ (°C) | 39 | 41,3 | 43,7 | 46 | 48,3 |

- Conditions liées à la cinétique.

  . Température $T_B$ : 41 °C.
  . Température $T_F$ : 14 °C.
  . Cinétique de refoidissement : T = f(t) :

| T (°C) | 41 | 31 | 31 | 21 | 21 | 14 |
|---|---|---|---|---|---|---|
| t (mn) | 0 | 15 | 30 | 45 | 50 | 60 |

  . Durée de l'état de sursaturation de la solution L soumise à cette cinétique : 70 minutes pour une vitesse d'agitation de 150 tours/mn.
  . Durée de cristallisation : 60 minutes.

- Conditions initiales.

  . Excès énantiomérique initial : 7,4 %.

| masse solvant (g) | masse (±) (g) | masse (+) (g) |
|---|---|---|
| 27,68 | 5,667 | 0,458 |

  . Durée du palier à $T_B$ : 30 minutes

. Vitesse d'agitation : 150 tours/mn au début de la cristallisation, puis 200 tours à la fin.

- Résultats.

| N° | masse antipode pur (g) | % pureté optique | Compensation sol. (±) 23,1 % |
|----|------------------------|------------------|------------------------------|
| 1 | 0,882 | (+) 93 | 1,085 |
| 2 | 0,914 | (-) 91 | 1,170 |
| 3 | 0,860 | (+) 93 | 1,224 |
| 4 | 0,887 | (-) 95 | 1,263 |
| 5 | 0,905 | (+) 96 | 1,100 |
| 6 | 0,929 | (-) 95 | 1,200 |
| 7 | 0,882 | (+) 93 | 1,888 |
| 8 | 1,002 | (-) 99 | 1,209 |
| 9 | 1,064 | (+) 93 | 1,387 |
| 10 | 1,164 | (-) 94 | 1,598 |
| 11 | 1,165 | (+) 90 | 2,356 |
| 12 | 1,071 | (-) 82 | 0,538 |

. Masse moyenne de cristaux d'antipode pur : 0,977 g.
. Excès énantiomérique moyen 0,489 g, soit 7,9 %.
. Pureté optique moyenne : 93 %.

8 - Dédoublement du 5-éthyl-5-phényl-hydantoïne.

Ce composé a été dédoublé par Cave et al. (Brevet des Etats-Unis No. 2 942 004), en utilisant la méthode conventionnelle SIPC dans un autre solvant moins approprié que le 2-méthoxy-éthanol.

a) Caractéristiques du produit.

- Température de fusion de l'antipode : 240 °C.
- Température de fusion du mélange racémique : 197°C.
- Pouvoir rotatoire spécifique à 20°C, c =1g/100ml, éthanol :

| $\lambda$(nm) | 589 | 578 | 546 | 436 | 365 |
|---------------|-----|-----|-----|-----|-----|
| $[\alpha]$ 20 (°) | 117 | 122 | 141 | 261 | 462 |

b) Dédoublement par la méthode AS3PC.

- Conditions liées aux équilibres.

. Solubilité des mélanges racémiques dans le 2-méthoxy-éthanol :

| T(°C) | 20 | 30 | 41 | 50 | 60 |
|-------|----|----|----|----|----|
| Solubilité s (% massique) | 13,6 | 15,6 | 17,0 | 19 | 23,0 |

. Solubilité de l'antipode R : 9,5 % à 41°C, rapport $\alpha$ = 1,8 à 41 °C.
. Coordonnées du point L : 17 % massique, température $T_L$ : 41 °C.
. Evolution de $T_{HOMO}$ avec l'excès énantiomérique (mélange racémique / (solvant + mélange racémique)) = 17 % = constante :

| % énantiomère | 0 | 2 | 4 | 6 | 8 |
|---|---|---|---|---|---|
| $T_{HOMO}$(°C) | 41 | 43,6 | 46,3 | 48,7 | 51,5 |

- <u>Conditions liées à la cinétique.</u>

  . Température $T_B$ : 44,5 °C.
  . Température $T_F$: 23 °C.
  . Cinétique de refoidissement : T = f(t) :

| T (°C) | 44,5 | 41 | 39 | 37 | 35 | 33 | 31 | 29 | 27 | 25 | 23 |
|---|---|---|---|---|---|---|---|---|---|---|---|
| t (mn) | 0 | 10 | 15 | 20 | 25 | 30 | 35 | 41 | 46 | 51 | 56 |

  . Durée de l'état de sursaturation de la solution L soumise à cette cinétique : 70 minutes pour une vitesse d'agitation de 275 tours/mn.
  . Durée de cristallisation : 56 minutes.

- <u>Conditions initiales.</u>

  . Excès énantiomérique initial : 6,4 %.

| masse solvant (g) | masse (±) (g) | masse (+) (g) |
|---|---|---|
| 27,666 | 5,666 | 0,3877 |

  . Durée du palier à $T_B$ : 30 minutes
  . Vitesse d'agitation : 200 tours/mn au début de la cristallisation, puis 275 tours à la fin.

- <u>Résultats</u>.

| N° | masse antipode pur (g) | % pureté optique | Compensation sol. (±) 23,1 % |
|---|---|---|---|
| 1 | 0,717 | (+) 91 | 0,742 |
| 2 | 0,838 | (-) 80 | 1,765 |
| 3 | 1,002 | (+) 92 | 0,894 |
| 4 | 1,016 | (-) 88 | 1,116 |
| 5 | 1,088 | (+) 92 | 0,806 |
| 6 | 1,161 | (-) 90 | 0,654 |
| 7 | 1,137 | (+) 86 | 1,446 |
| 8 | 1,135 | (-) 85 | 2,440 |
| 9 | 1,179 | (+) 87 | |

  . Masse moyenne de cristaux d'antipode pur : 1,030 g.
  . Excès énantiomérique moyen 0,515 g, soit 8,5 %.
  . Pureté optique moyenne : 88 %.

9 - <u>Dédoublement du 5-méthyl,5-(4-chlorophényl)-hydantoïne.</u>

  a) <u>Caractéristiques du produit.</u>

- Température de fusion de l'antipode : 305°C.
- Température de fusion du mélange racémique : 263°C.
- Pouvoir rotatoire spécifique à 20 °C, c = 1g/100ml, éthanol :

| λ(nm) | 578 | 546 | 436 | 365 |
|---|---|---|---|---|
| $[\alpha]$ 20 (°) | 112 | 130 | 241 | 434 |

b) Dédoublement par la méthode AS3PC.

- <u>Conditions liées aux équilibres.</u>

  . Solubilité des mélanges racémiques dans le 2-méthoxy-éthanol :

| T(°C) | 20 | 40 | 50 | 60 |
|---|---|---|---|---|
| Solubilité s (% massique) | 3,5 | 4,7 | 5,6 | 6,5 |

  . Solubilité de l'antipode R : 1,72 % à 20°C, rapport $\alpha = 2,03$ à 20 °C.
  . Coordonnées du point L : 6,05 % massique, température $T_L$ : 55 °C.
  . Evolution de $T_{HOMO}$ avec l'excès énantiomérique (mélange racémique / (solvant + mélange racémique)) = 6,05 % = constante :

| % énantiomère | 0 | 4 | 8 |
|---|---|---|---|
| $T_{HOMO}$ (°C) | 55 | 60 | 65 |

- <u>Conditions liées à la cinétique.</u>

  . Température $T_B$ : 57°C.
  . Température $T_F$ : 27°C.
  . Cinétique de refoidissement : T = f(t) :

| T (°C) | 57 | 35 | 27 | 27 |
|---|---|---|---|---|
| t (mn) | 0 | 32 | 52 | 115 |

  . Durée de l'état de sursaturation de la solution L soumise à cette cinétique : 120 minutes pour une vitesse d'agitation de 150 tours/mn.
  . Durée de cristallisation : 115 minutes.

- <u>Conditions initiales.</u>

  . Excès énantiomérique initial : 6,52 %.

| masse solvant (g) | masse (±) (g) | masse (+) (g) |
|---|---|---|
| 95,7 | 6,166 | 0,430 |

  . Durée du palier à $T_B$ : 60 minutes,
  . Vitesse d'agitation : 200 tours/mn.

- <u>Résultats.</u>

| N° | masse antipode pur (g) | % pureté optique | Compensation sol. (±) 23,1 % |
|---|---|---|---|
| 1 | 0,889 | (+) 90,7 | 2,010 |
| 2 | 0,931 | (-) 91,8 | 1,826 |
| 3 | 0,661 | (+) 91,1 | 1,992 |
| 4 | 1,083 | (-) 89,0 | 2,258 |

(suite)

| N° | masse antipode pur (g) | % pureté optique | Compensation sol. (±) 23,1 % |
|---|---|---|---|
| 5 | 1,381 | (+) 92,9 | 5,559 |
| 6 | 1,330 | (-) 92,4 | 2,535 |
| 7 | 1,397 | (+) 91,9 | 2,195 |
| 8 | 1,410 | (-) 93,9 | |

- Masse moyenne de cristaux d'antipode pur : 1,13 g.
- Excès énantiomérique moyen 0,568 g, soit 8,6 %.
- Pureté optique moyenne : 91,7 %.

10 - - Dédoublement du thréitol.

a) Caractéristiques physiques du produit.

- Température de fusion de l'antipode : 92°C.
- Température de fusion du mélange racémique : 70°C.
- Pouvoir rotatoire spécifique à 20°C, c = 1g/100ml, éthanol :

| $\lambda$(nm) | 589 | 578 | 546 | 436 | 365 |
|---|---|---|---|---|---|
| $[\alpha]20(°)$ | 14,2 | 14,8 | 16,8 | 29,3 | 47,4 |

b) Dédoublement par la méthode AS3PC.

- Conditions liées aux équilibres.

- Solubilité du mélange racémique dans le mélange éthanol 95 % / eau 5 % :

| T(°C) | 21,1 | 23,1 | 25,1 | 26,7 | 27,0 | 30,9 | 31,4 | 34,3 |
|---|---|---|---|---|---|---|---|---|
| Solubilité s (% massique) | 9,3 | 10,2 | 11,3 | 12,2 | 12,5 | 15,9 | 16,6 | 20,1 |

- Solubilité de l'antipode R : 4,4 % à 27 °C, rapport $\alpha = 2,8$ à 27 °C.
- Coordonnées du point L : 12,5 % massique, température $T_L$ : 27 °C.
- Evolution de $T_{HOMO}$ avec l'excès énantiomérique (mélange racémique / (solvant + mélange racémique)) = 12,5 % = constante :

| % énantiomère | 0 | 2 | 4,12 | 5, 85 | 7, 90 |
|---|---|---|---|---|---|
| $T_{HOMO}(°C)$ | 27,0 | 27,8 | 28,6 | 29,3 | 29,8 |

- Conditions liées à la cinétique.

- Température $T_B$ : 27,5 °C.
- Température $T_F$ : 19,4 °C.
- Cinétique de refoidissement : T = f(t) :

| T (°C) | 27,5 | 26,8 | 26,8 | 23,3 | 23,3 | 19,4 | 19,4 |
|---|---|---|---|---|---|---|---|
| t (mn) | 0 | 1,5 | 5 | 11 | 15 | 22 | 60 |

- Durée de l'état de sursaturation de la solution L soumise à cette cinétique : 60 minutes pour une vitesse d'agitation de 100 tours/mn.
- Durée de cristallisation : 60 minutes.

- Conditions initiales.

    . Excès énantiomérique initial : 6 %.

| masse solvant (g) | masse (±) (g) | masse (+) (g) |
|---|---|---|
| 87,50 | 12,50 | 0,800 |

    . Durée du palier à $T_B$ : 20 minutes
    . Vitesse d'agitation : 100 tours/mn au début de la cristallisation, puis 120 tours/mn à la fin.

- Résultats.
    La filtration est réalisée sur un verre fritté N°2; les cristaux sont lavés avec du diisopropyl ether.

| N° | masse antipode pur (g) | % pureté optique | Compensation sol. (±) 23,1 % |
|---|---|---|---|
| 1 | 1,637 | (+) 89 | 2,107 |
| 2 | 1,740 | (-) 96,2 | 2,260 |
| 3 | 1,916 | (+) 96,7 | 2,364 |
| 4 | 2,040 | (-) 96 | 2,622 |
| 5 | 1,703 | (+) 93,3 | 2,353 |
| 6 | 1,856 | (-) 96,2 | 2,387 |
| 7 | 1,932 | (+) 96 | 2,403 |
| 8 | 1,649 | (-) 93,3 | 2,39 |
| 9 | 1,615 | (+) 93,2 | 2,17 |
| 10 | 1,721 | (-) 92,4 | 2,39 |

    . Masse moyenne de cristaux d'antipode pur : 1,780 g.
    . Excès énantiomérique moyen 0,890 g, soit 6,7 %.
    . Pureté optique moyenne : 94,2 %.

**Revendications**

1. Procédé de dédoublement de deux énantiomères optiques par cristallisation préférentielle, caractérisé en ce qu'il comprend les étapes suivantes :

    a) on réalise un ensemble composé du mélange racémique de cristaux sous forme de conglomérat, du premier énantiomère et de solvant, dont le point figuratif E, défini par les variables concentration et température $T_B$, se situe dans le domaine biphasé du premier énantiomère en excès, et est en équilibre avec sa solution saturée;
    b) on applique une loi de programmation de l'abaissement de température au mélange biphasé préparé à l'étape (a), ladite loi de programmation étant telle que les liqueurs-mères gardent une faible sursaturation qui privilégie la croissance de l'énantiomère présent sous forme de cristaux, tout en interdisant la nucléation spontanée du second énantiomère présent dans la solution;
    c) on adapte pendant toute la durée de la croissance cristalline de l'étape (b) une vitesse d'agitation légèrement croissante en fonction du temps de façon à ce que celle-ci soit à tout moment, suffisamment lente pour favoriser une croissance du premier énantiomère en évitant de générer des forces de striction trop importantes provoquant une nucléation non maîtrisée, et suffisamment rapide pour réaliser une suspension homogène et un renouvellement rapide de la liqueur-mère autour de chaque cristallite du premier énantiomère;
    d) on récolte les cristaux du premier énantiomère;
    e) on additionne aux liqueurs-mères résultant de la récolte réalisée à l'étape (d), le mélange racémique de cristaux sous forme de conglomérat, et on porte le nouvel ensemble à un palier de température $T_B$ pendant la durée nécessaire à l'obtention de l'équilibre thermodynamique de sorte que le point figuratif E' soit symétrique de E par rapport au plan des mélanges racémiques du système solvant, antipode (-), antipode (+), ledit point E' se situant dans le domaine biphasé du second énantiomère en excès et en équilibre avec sa solution saturée;

f) on applique la même loi de programmation de l'abaissement de température qu'à l'étape (b), au mélange biphasé préparé à l'étape (e) contenant le second énantiomère, de sorte que les liqueurs-mères gardent une faible sursaturation pendant la cristallisation afin de privilégier la croissance de l'énantiomère présent sous forme de cristaux tout en interdisant la nucléation spontanée du premier énantiomère présent dans la solution;

g) on adapte pendant toute la durée de la croissance cristalline de l'étape f), une vitesse d'agitation légèrement croissante en fonction du temps de façon à ce que celle-ci soit, à tout moment, suffisamment lente pour favoriser la croissance du second énantiomère en évitant de générer des forces de striction trop importantes provoquant une nucléation non maîtrisée, et suffisamment rapide pour obtenir une suspension homogène et un renouvellement rapide de la liqueur-mère autour de chaque cristallite du second énantiomère;

h) on récolte les cristaux du second énantiomère;

i) on additionne aux liqueurs-mères résultant de la récolte cristalline réalisée à l'étape (h), le mélange racémique de cristaux sous forme de conglomérat, pour obtenir un ensemble dont la composition est identique à celle de l'ensemble E initial;

j) on répète les étapes (a), (b), (c), (d), (e), (f), (g), (h) et (i) pour obtenir successivement le premier puis le second des deux énantiomères.

2. Procédé selon la revendication 1, caractérisé en ce qu'à l'étape (a) le choix du ou des solvants et de la gamme de température de travail sont définis de façon à avoir simultanément :

- des antipodes qui réalisent un conglomérat et dont l'éventuel racémate est métastable dans la gamme de température de travail;
- des liqueurs suffisamment concentrées mais de faible viscosité et de faible tension de vapeur;
- une absence de solvolyse et de racémisation;
- une stabilité des solvates si ceux-ci sont présents à l'équilibre et s'il s'agit d'énantiomères dédoublables.

3. Procédé selon l'une quelconque des revendications 1 et 2, caractérisé en ce qu'aux étapes (a) et (e), la température $T_B$ est supérieure à la température $T_L$ d'homogénéisation de la quantité de mélange racémique contenue dans la suspension initiale, et en ce que, à partir de la courbe de variation de $T_{HOMO}$ en fonction de l'excès énantiomérique et pour une concentration constante en mélange racémique $X_L$, ladite température $T_B$ est définie de façon à ce que la masse de fins cristaux du premier énantiomère des étapes (a) et (i) et du second énantiomère de l'étape (e), en équilibre avec leur solution saturée, représente au maximum 50 % et de préférence entre environ 25 et 40 % de la récolte attendue

4. Procédé selon l'une quelconque des revendications précédentes, caractérisé en ce qu'aux étapes (b) et (f), la loi de programmation de l'abaissement de température de $T_B$ à $T_F$ adaptée au montage expérimental, est définie de façon :

- à obtenir une faible sursaturation pendant toute la durée de la cristallisation de l'énantiomère présent sous forme de cristaux au début de chaque cycle, cette faible sursaturation provoquant une croissance et une nucléation secondaires douces;
- à atteindre à $T_F$ le maximum de sursaturation de l'autre énantiomère sans nucléation primaire,
- à obtenir une récolte en cristaux aux étapes (d) et (h) qui, après addition de mélange racémique et compensation aux étapes (e) et (i), permet la cyclicité des opérations.

5. Procédé selon la revendication 4, caractérisé en ce que la loi de programmation de l'abaissement de température est déterminée pour sa partie de $T_L$ à $T_F$ par refroidissement de la solution de concentration $X_L$ de $T_L + 1°C$ à $T_F$, $T_F$ étant inférieur à $T_L - (T_{HOMO} - T_L)$, afin d'obtenir une solution sursaturée sans nucléation primaire tout en permettant une récolte double de l'excès énantiomérique initial, et en ce que ladite loi de programmation de l'abaissement de température est déterminée pour sa partie de $T_B$ à $T_L$ par extrapolation de cette même loi déterminée de $T_L + 1°C$ à $T_F$.

6. Procédé selon l'une quelconque des revendications précédentes, caractérisé en ce qu'aux étapes (b) et (f), la thermicité accompagnant le dépôt du premier énantiomère et du second énantiomère est intégrée dans la loi de programmation de l'abaissement de température.

7. Procédé selon l'une quelconque des revendications précédentes, caractérisé en ce qu'aux étapes (e) et (i), on effectue des compensations en solvant.

8. Procédé selon l'une quelconque des revendications précédentes, caractérisé en ce qu'aux étapes (a), (e) et (i), les fins cristaux du mélange racémique sous forme de conglomérat qui sont ajoutés, ont subi avant d'être introduits, un traitement préalable accélérant l'étape de dissolution, tel que un broyage et un tamisage, un traitement par des ondes ultra-sonores, une lyophilisation partielle.

9. Procédé selon l'une quelconque des revendications précédentes, caractérisé en ce qu'aux étapes (a), (e) et (i), on élève la vitesse d'agitation.

10. Procédé de dédoublement des deux énantiomères optiques du tartrate double de sodium et d'ammonium tétrahydraté selon les revendications 1 à 9, caractérisé en ce que :

   - à l'étape (a), le mélange initial est le suivant :

      . solvant : 5,67 g d'eau,
      . mélange racémique : 4,83 g,
      . antipode (+) : 0,23 g, et,
      . $T_L$ = 16,3 °C,
      . TB = 17,3 °C,
      . durée du palier à $T_B$ = 40 mn

   - à l'étape (b), la loi de programmation de l'abaissement de température de $T_B$ à $T_F$ est donnée par les valeurs des températures en fonction du temps ci-dessous :

| T (°C) | 17,3 | 17,0 | 16,5 | 15,4 | 14,0 | 13,0 | 12,1 | 12,0 | 12,0 |
|--------|------|------|------|------|------|------|------|------|------|
| t (mn) | 0 | 3 | 5 | 8 | 12,5 | 16 | 20 | 25 | 30 |

   - à l'étape (c), dans le montage expérimental utilisé, l'agitation est de 150 tours/mn au début et 170 tours/mn à la fin de la cristallisation.
   - à l'étape (d), la récolte des cristaux est effectuée sur verre fritté n°2.
   - à l'étape (e), on ajoute du mélange racémique broyé et tamisé à 250 µ, et en plus des compensations des pertes habituelles, on ajoute de l'ammoniaque pour maintenir un léger excès en ce constituant, et :

      . $T_L$ = 16,3 °C,
      . $T_B$ = 17,3 °C,
      . durée du palier à $T_B$ = 40 mn

   - à l'étape (f), la loi de programmation de l'abaissement de température de $T_B$ à $T_F$ est donnée par les valeurs des températures en fonction du temps ci-dessous :

| T (°C) | 17,3 | 17,0 | 16,5 | 15,4 | 14,0 | 13,0 | 12,1 | 12,0 | 12,0 |
|--------|------|------|------|------|------|------|------|------|------|
| t (mn) | 0 | 3 | 5 | 8 | 12,5 | 16 | 20 | 25 | 30 |

   - à l'étape (g), dans le montage expérimental utilisé, l'agitation est de 150 tours/mn au début et 170 tours/mn à la fin de la cristallisation.
   - à l'étape (h), la récolte des cristaux est effectuée sur verre fritté n°2.
   - à l'étape (i), on ajoute du mélange racémique broyé et tamisé à 250 µ et, en plus des compensations des pertes habituelles, on ajoute de l'ammoniaque pour maintenir un léger excès en ce constituant.

11. Procédé de dédoublement des deux énantiomères optiques de la 5-méthyl-5-phényl-hydantoïne selon les revendications 1 à 9, caractérisé en ce que :

   - à l'étape (a), le mélange initial est le suivant :

- solvant : 15,704 g de 2-méthoxy-éthanol,
- mélange racémique : 4,296 g,
- antipode (+) : 0,229 g,

et,

- $T_L = 37\ °C$,
- $T_B = 40\ °C$,
- durée du palier à $T_B = 30$ mn;

- à l'étape (b), la loi de programmation de l'abaissement de température de $T_B$ à $T_F$ est donnée par les valeurs des températures en fonction du temps ci-dessous :

| T (°C) | 40 | 35 | 30 | 25 | 20 | 20 |
|--------|----|----|----|----|----|----|
| t (mn) | 0  | 12 | 22 | 30 | 38 | 46 |

- à l'étape (c), dans le montage expérimental utilisé, l'agitation est de 100 tours/mn au début et 150 tours/mn à la fin de la cristallisation;
- à l'étape (d), la récolte des cristaux est effectuée sur verre fritté n°3;
- à l'étape (e), on ajoute du mélange racémique,. on effectue les compensations habituelles, et :

- $T_L = 37\ °C$,
- $T_B = 40\ °C$,
- durée du palier à $T_B = 30$ mn

- à l'étape (f), la loi de programmation de l'abaissement de température de $T_B$ à $T_F$ est donnée par les valeurs des températures en fonction du temps ci-dessous :

| T (°C) | 40 | 35 | 30 | 25 | 20 | 20 |
|--------|----|----|----|----|----|----|
| t (mn) | 0  | 12 | 22 | 30 | 38 | 46 |

- à l'étape (g), dans le montage expérimental utilisé, l'agitation est de 100 tours/mn au début et 150 tours/mn à la fin de la cristallisation.
- à l'étape (h), la récolte des cristaux est effectuée sur verre fritté n°3.
- à l'étape (i), on ajoute du mélange racémique et on effectue les compensations nécessaires.

12. Procédé de dédoublement des deux énantiomères optiques de la 5-méthyl-5-(4-méthyl phényl)-hydantoïne selon les revendications 1 à 9, caractérisé en ce que :

- à l'étape (a), le mélange initial est le suivant :

- solvant : 27,68 g de 2-méthoxy-éthanol,
- mélange racémique : 5,667 g,
- antipode (+) : 0,458 g,

et,

- $T_L = 39\ °C$,
- $TB = 41\ °C$,
- durée du palier à $T_B = 30$ mn;

- à l'étape (b), la loi de de l'abaissement de température de $T_B$ à $T_F$ est donnée par les valeurs des températures en fonction du temps ci-dessous :

| T (°C) | 41 | 31 | 31 | 21 | 21 | 14 |
|--------|----|----|----|----|----|----|

(suite)

| t (mn) | 0 | 15 | 30 | 45 | 50 | 60 |
|--------|---|----|----|----|----|----|

- à l'étape (c), dans le montage expérimental utilisé, l'agitation est de 150 tours/mn au début et 200 tours/mn à la fin de la cristallisation;
- à l'étape (d), la récolte des cristaux est effectuée sur verre fritté n°3;
- à l'étape (e), on ajoute du mélange racémique, on effectue les compensations habituelles, et :

  . $T_L = 39\ °C$,
  . TB = 41 °C,
  . durée du palier à $T_B$ = 30 mn

- à l'étape (f), la loi de programmation de l'abaissement de température de $T_B$ à $T_F$ est donnée par les valeurs des températures en fonction du temps ci-dessous :

| T (°C) | 41 | 31 | 31 | 21 | 21 | 14 |
|--------|----|----|----|----|----|----|
| t (mn) | 0 | 15 | 30 | 45 | 50 | 60 |

- à l'étape (g), dans le montage expérimental utilisé, l'agitation est de 150 tours/mn au début et 200 tours/mn à la fin de la cristallisation.
- à l'étape (h), la récolte des cristaux est effectuée sur verre fritté n°3.
- à l'étape (i), on ajoute du mélange racémique et on effectue les compensations nécessaires.

13. Procédé de dédoublement des deux énantiomères optiques de la 5-éthyl-5-phényl-hydantoïne selon les revendications 1 à 9, caractérisé en ce que :

- à l'étape (a), le mélange initial est le suivant :

  . solvant : 27,666 g de 2-méthoxy-éthanol,
  . mélange racémique : 5,666 g,
  . antipode (+) : 0,3877 g,

  et,

  . $T_L = 41\ °C$,
  . TB = 44,5 °C,
  . durée du palier à $T_B$ = 30 mn;

- à l'étape (b), la loi de programmation de l'abaissement de température de $T_B$ à $T_F$ est donnée par les valeurs des températures en fonction du temps ci-dessous :

| T (°C) | 44,5 | 41 | 39 | 37 | 35 | 33 | 31 | 29 | 27 | 25 | 23 |
|--------|------|----|----|----|----|----|----|----|----|----|----|
| t (mn) | 0 | 10 | 15 | 20 | 25 | 30 | 35 | 41 | 46 | 51 | 56 |

- à l'étape (c), dans le montage expérimental utilisé, l'agitation est de 200 tours/mn au début et 275 tours/mn à la fin de la cristallisation;
- à l'étape (d), la récolte des cristaux est effectuée sur verre fritté n°3;
- à l'étape (e), on ajoute du mélange racémique, on effectue les compensations habituelles, et :

  . $T_L = 41\ °C$,
  . TB = 44,5 °C,
  . durée du palier à $T_B$ = 30 mn

- à l'étape (f), la loi de programmation de l'abaissement de température de $T_B$ à $T_F$ est donnée par les valeurs des températures en fonction du temps ci-dessous :

| T (°C) | 44,5 | 41 | 39 | 37 | 35 | 33 | 31 | 29 | 27 | 25 | 23 |
|---|---|---|---|---|---|---|---|---|---|---|---|
| t (mn) | 0 | 10 | 15 | 20 | 25 | 30 | 35 | 41 | 46 | 51 | 56 |

- à l'étape (g), dans le montage expérimental utilisé, l'agitation est de 200 tours/mn au début et 275 tours/mn à la fin de la cristallisation.
- à l'étape (h), la récolte des cristaux est effectuée sur verre fritté n°3.
- à l'étape (i), on ajoute du mélange racémique et on effectue les compensations nécessaires.

14. Procédé de dédoublement des deux énantiomères optiques de la 5-méthyl-5-(4-chlorophényl)-hydantoïne selon les revendications : 9, caractérisé en ce que :

- à l'étape (a), le mélange initial est le suivant :

  - solvant : 95,7 g de 2-méthoxy-éthanol,
  - mélange racémique : 6,166 g,
  - antipode (+) : 0,430 g,

et,

  - $T_L = 55\ °C$,
  - $TB = 57\ °C$,
  - durée du palier à $T_B = 60$ mn;

- à l'étape (b), la loi de programmation de l'abaissement de température de $T_B$ à $T_F$ est donnée par les valeurs des températures en fonction du temps ci-dessous:

| T (°C) | 57 | 35 | 27 | 27 |
|---|---|---|---|---|
| t (mn) | 0 | 32 | 52 | 115 |

- à l'étape (c), dans le montage expérimental utilisé, l'agitation est de 150 tours/mn au début et 200 tours/mn à la fin de la cristallisation;
- à l'étape (d), la récolte des cristaux est effectuée sur verre fritté n°3;
- à l'étape (e), on ajoute du mélange racémique, on effectue les compensations habituelles, et :

  - $T_L = 55\ °C$,
  - $TB = 57\ °C$,
  - durée du palier à $T_B = 60$ mn

- à l'étape (f), la loi de programmation de l'abaissement de température de $T_B$ à $T_F$ est donnée par les valeurs des températures en fonction du temps ci-dessous :

| T (°C) | 57 | 35 | 27 | 27 |
|---|---|---|---|---|
| t (mn) | 0 | 32 | 52 | 115 |

- à l'étape (g), dans le montage expérimental utilisé, l'agitation est de 200 tours/mn au début et 275 tours/mn à la fin de la cristallisation.
- à l'étape (h), la récolte des cristaux est effectuée sur verre fritté n°3.
- à l'étape (i), on ajoute du mélange racémique et on effectue les compensations nécessaires.

15. Procédé de dédoublement des deux énantiomères optiques du thréitol selon les revendications 1 à 9, caractérisé en ce que :

- à l'étape (a), le mélange initial est le suivant :

- solvant : 87,5 g de mélange éthanol 95 % / eau 5 %,
- mélange racémique : 12,50 g,
- antipode (+) : 0,800 g,

et,

- $T_L$ = 27 °C,
- $T_B$ = 27,5 °C,
- durée du palier à $T_B$ = 20 mn;

- à l'étape (b), la loi de programmation de l'abaissement de température de $T_B$ à $T_F$ est donnée par les valeurs des températures en fonction du temps ci-dessous :

| T (°C) | 27,5 | 26,8 | 26,8 | 23,3 | 23,3 | 19,4 | 19,4 |
|--------|------|------|------|------|------|------|------|
| t (mn) | 0 | 1,5 | 5 | 11 | 15 | 22 | 60 |

- à l'etape (c), dans le montage expérimental utilisé, l'agitation est de 100 tours/mn au début et 120 tours/mn à la fin de la cristallisation;
- à l'étape (d), la récolte des cristaux est effectuée sur verre fritté n°2 suivie d'un lavage avec du diisopropyléther;
- à l'étape (e), on ajoute du mélange racémique, on effectue les compensations habituelles, et :

- $T_L$ = 27 °C,
- TB = 27,5 °C,
- durée du palier à $T_B$ = 20 mn

- à l'étape (f), la loi de programmation de l'abaissement de température de $T_B$ à $T_F$ est donnée par les valeurs des températures en fonction du temps ci-dessous :

| T (°C) | 27,5 | 26,8 | 26,8 | 23,3 | 23,3 | 19,4 | 19,4 |
|--------|------|------|------|------|------|------|------|
| t (mn) | 0 | 1,5 | 5 | 11 | 15 | 22 | 60 |

- à l'étape (g), dans le montage expérimental utilisé, l'agitation est de 100 tours/mn au début et 120 tours/mn à la fin de la cristallisation.
- à l'étape (h), la récolte des cristaux est effectuée sur verre fritté n°2 suivie d'un lavage avec du diisopropyléther.
- à l'étape (i), on ajoute du mélange racémique et on effectue les compensations nécessaires.

**Patentansprüche**

1. Verfahren zur Spaltung zweier optischer Enantiomere durch fortschreitende Kristallisierung, dadurch gekennzeichnet, daß es folgende Schritte umfaßt:

   a) es wird ein klumpenförmiger, aus dem razemischen Kristallgemisch bestehender Komplex aus dem ersten Enantiomer und Lösemittel erstellt, dessen durch die Variablen Konzentration und Temperatur $T_B$ definierter figurativer Punkt E sich überschüssig im zweiphasigen Bereich des ersten Enantiomers befindet und der mit seiner gesättigten Lösung im Gleichgewicht steht;
   b) es wird ein Programmiergesetz zur Temperatursenkung auf das zweiphasige im ersten Schritt (a) zubereitete Gemisch angewandt, wobei das besagte Programmiergesetz so ausgelegt ist, daß die Stammlösungen eine geringe Übersättigung beibehalten, die das Wachstum des in Form von Kristallen vorliegenden Enantiomers begünstigt und dabei die spontane Nukleation des zweiten in der Lösung vorhandenen Enantiomers unterbindet;
   c) während der gesamten Dauer des Kristallwachstums von Schritt (b) wird eine zeitabhängig leicht ansteigende Rührgeschwindigkeit angepaßt, so daß diese jederzeit langsam genug ist, um das Wachstum des ersten Enantiomers zu fördern und dabei zu vermeiden, zu große Zusammenziehungskräfte zu generieren, die eine unkontrollierte Nukleation hervorrufen, und schnell genug ist, um eine gleichmäßige Suspension und eine schnelle Erneuerung der Stammlösung um den jeweiligen Kristallit des ersten Enantiomers zu erstellen;

33

d) die Kristalle des ersten Enantiomers werden gesammelt;

e) zu den aus der bei Schritt (d) realisierten Ausbeute hervorgehenden Stammlösungen wird das klumpenförmige razemische Kristallgemisch hinzugefügt, und der neue Komplex wird für eine zur Erlangung des thermodynamischen Gleichgewichts notwendige Dauer auf eine Temperaturstufe $T_B$ gebracht, so daß der figurative Punkt E' zu E symmetrisch ist im Verhältnis zur Ebene der razemischen Gemische des Systems Lösemittel, (-)Antipode, (+)Antipode, wobei der besagte Punkt E' sich überschüssig im zweiphasigen Bereich des zweiten Enantiomers und im Gleichgewicht mit seiner gesättigten Lösung befindet;

f) es wird dasselbe Programmiergesetz zur Temperatursenkung wie bei Schritt (b) auf das zweiphasige im Schritt (e) zubereitete Gemisch, das den zweiten Enantiomer enthält, angewandt, so daß die Stammlösungen eine leichte Übersättigung während der Kristallisierung beibehalten, um das Wachstum des in Kristallform vorliegenden Enantiomers zu begünstigen und dabei die spontane Nukleation des ersten in der Lösung vorhandenen Enantiomers zu unterbinden;

g) während der gesamten Dauer des Kristallwachstums von Schritt (f) wird eine zeitabhängig leicht ansteigende Rührgeschwindigkeit angepaßt, so daß diese jederzeit langsam genug ist, um das Wachstum des zweiten Enantiomers zu fördern und dabei zu vermeiden, zu große Zusammenziehungskräfte zu generieren, die eine unkontrollierte Nukleation hervorrufen, und schnell genug ist, um eine gleichmäßige Suspension und eine schnelle Erneuerung der Stammlösung um den jeweiligen Kristallit des zweiten Enantiomers zu erstellen;

h) die Kristalle des zweiten Enantiomers werden gesammelt;

i) zu den aus der bei Schritt (h) realisierten Kristallausbeute hervorgehenden Stammlösungen wird das klumpenförmige razemische Kristallgemisch hinzugefügt, um einen Komplex zu erhalten, dessen Zusammenstellung mit der des ursprünglichen Komplexes E identisch ist;

j) die Schritte (a), (b), (c), (d), (e), (f), (g), (h) und (i) werden wiederholt, um nacheinander das erste und dann das zweite der beiden Enantiomere zu erhalten.

2. Verfahren nach Patentanspruch 1, dadurch gekennzeichnet, daß im Schritt (a) die Wahl des oder der Lösemittel und des Arbeitstemperaturbereichs so definiert ist, daß man gleichzeitig:

- Antipoden erhält, die einen Klumpen bilden und deren eventuelles Razemat im Arbeitstemperaturbereich metastabil ist;
- Flüssigkeiten erhält, die genügend konzentriert sind, aber eine geringe Viskosität und geringe Dampfspannung haben;
- keine Solvolyse und Razemisierung erhält;
- Stabilität der Solvate erhält, falls diese beim Gleichgewicht vorhanden sind und falls es sich um spaltbare Enantiomere handelt.

3. Verfahren nach einem der Patentansprüche 1 und 2, dadurch gekennzeichnet, daß bei den Schritten (a) und (e) die Temperatur $T_B$ höher als die Temperatur $T_L$ für die Homogenisierung der in der ursprünglichen Suspension enthaltenen razemischen Gemischmenge ist, und dadurch daß, ausgehend von der Variationskurve von $T_{HOMO}$, abhängig vom enantiomerischen Überschuß und bei einer konstanten Konzentration des razemischen Gemischs $X_L$, die besagte Temperatur $T_B$ so definiert ist, daß die Masse der Feinkristalle des ersten Enantiomers der Schritte (a) und (i) und des zweiten Enantiomers des Schrittes (e) im Gleichgewicht mit ihrer gesättigten Lösung höchstens 50% und möglichst zwischen ungefähr 25 und 40% der erwarteten Ausbeute darstellt.

4. Verfahren nach einem der vorhergehenden Patentansprüche, dadurch gekennzeichnet, daß bei den Schritten (b) und (f) das sich dem Versuchsaufbau anpassende Programmiergesetz zur Temperatursenkung von $T_B$ auf $T_F$, so definiert ist, daß man:

- eine geringe Übersättigung während der gesamten Dauer der Kristallisierung des am Anfang jedes Zyklus kristallförmig vorliegenden Enantiomers erhält, wobei diese geringe Übersättigung sanftes sekundäres Wachstum und Nukleation hervorruft;
- bei $T_F$ die höchste Übersättigung des anderen Enantiomers ohne primäre Nukleation erreicht;
- eine Kristallausbeute bei den Schritten (d) und (h) erhält, die nach Hinzufügen eines razemischen Gemischs und Ausgleich bei den Schritten (e) und (i) die regelmäßige Wiederholung der Vorgänge erlaubt.

5. Verfahren nach Patentanspruch 4, dadurch gekennzeichnet, daß das Programmiergesetz zur Temperatursenkung für seinen Anteil von $T_L$ auf $T_F$ durch Abkühlung der Konzentrationslösung $X_L$ von $T_L$ + 1°C auf $T_F$ festgelegt ist, wobei $T_F$ kleiner als $T_L$ - ($T_{HOMO}$ - $T_L$) ist, um eine übersättigte Lösung ohne primäre Nukleation zu erhalten und dabei eine doppelte Ausbeute des ursprünglichen enantiomerischen Überschußes zu ermöglichen, und dadurch daß das besagte Programmiergesetz zur Temperatursenkung für seinen Anteil von $T_B$ auf $T_L$ durch Extrapolation desselben festgelegten Programmiergesetz $T_L$ + 1°C auf $T_F$ festgelegt ist.

6. Verfahren nach einem der vorhergehenden Patentansprüche, dadurch gekennzeichnet, daß bei den Schritten (b) und (f) die mit der Ausscheidung des ersten Enantiomers und des zweiten Enantiomers verbundene Thermizität in das Programmiergesetz zur Temperatursenkung integriert ist.

7. Verfahren nach einem der vorhergehenden Patentansprüche, dadurch gekennzeichnet, daß bei den Schritten (e) und (i) Lösemittelausgleiche vorgenommen verden.

8. Verfahren nach einem der vorhergehenden Patentansprüche, dadurch gekennzeichnet, daß bei den Schritten (a), (e) und (i) die hinzugefügten Feinkristalle des klumpenförmigen razemischen Gemischs, bevor sie zugeführt werden, eine Vorbehandlung erhalten, die den Auflösungsschritt beschleunigt, wie z.B. Zerkleinerung und Siebung, Ultraschallwellen-Behandlung, Teilgefriertrocknung.

9. Verfahren nach einem der vorhergehenden Patentansprüche, dadurch gekennzeichnet, daß bei den Schritten (a), (e) und (i) die Rührgeschwindigkeit angehoben wird.

10. Verfahren zur Spaltung der beiden optischen Enantiomere von Seignettesalz und Ammonium Tetrahydrat nach den Patentansprüchen 1 bis 9, dadurch gekennzeichnet, daß:

   - bei Schritt (a) das ursprüngliche Gemisch folgendes ist:

      . Lösemittel: 5,67 g Wasser
      . razemisches Gemisch: 4,83 g
      . (+)Antipode: 0,23 g und
      . $T_L$ = 16,3°C
      . $T_B$ = 17,3°C
      . Dauer der $T_B$-Stufe = 40 min

   - bei Schritt (b) das Programmiergesetz zur Temperatursenkung von $T_B$ auf $T_F$ durch die nachstehenden zeitabhängigen Temperaturwerte gegeben ist:

| T (°C) | 17,3 | 17,0 | 16,5 | 15,4 | 14,0 | 13,0 | 12,1 | 12,0 | 12,0 |
|--------|------|------|------|------|------|------|------|------|------|
| t (min) | 0 | 3 | 5 | 8 | 12,5 | 16 | 20 | 25 | 30 |

   - bei Schritt (c) für den verwendeten Versuchsaufbau das Rühren am Anfang 150 Umdrehungen/min und am Ende der Kristallisierung 170 Umdrehungen/min beträgt.
   - bei Schritt (d) die Ausbeute der Kristalle auf Sinterglas Nr. 2 vorgenommen wird.
   - bei Schritt (e) ein auf 250 μ zerkleinertes und gesiebtes razemisches Gemisch hinzugefügt wird, und außer den Ausgleichen für normale Verluste Ammonium hinzugefügt wird, um für diesen Bestandteil einen geringfügigen Überschuß beizubehalten, und:

      . $T_L$ = 16,3°C
      . $T_B$ = 17,3°C
      . Dauer der $T_B$-Stufe = 40 min

   - bei Schritt (f) das Programmiergesetz zur Temperatursenkung von $T_B$ auf $T_F$ durch die nachstehenden zeitabhängigen Temperaturwerte gegeben ist:

| T (°C) | 17,3 | 17,0 | 16,5 | 15,4 | 14,0 | 13,0 | 12,1 | 12,0 | 12,0 |
|--------|------|------|------|------|------|------|------|------|------|
| t (min) | 0 | 3 | 5 | 8 | 12,5 | 16 | 20 | 25 | 30 |

   - bei Schritt (g) für den verwendeten Versuchsaufbau das Rühren am Anfang 150 Umdrehungen/min und am Ende der Kristallisierung 170 Umdrehungen/min beträgt.
   - bei Schritt (h) die Ausbeute der Kristalle auf Sinterglas Nr. 2 vorgenommen wird.

- bei Schritt (i) ein auf 250 µ zerkleinertes und gesiebtes razemisches Gemisch hinzugefügt wird, und außer den Ausgleichen für normale Verluste Ammonium hinzugefügt wird, um für diesen Bestandteil einen geringfügigen Überschuß beizubehalten.

11. Verfahren zur Spaltung der beiden optischen Enantiomere von 5-Methyl-5-Phenyl-Hydantoin nach den Patentansprüchen 1 bis 9, dadurch gekennzeichnet, daß:

- bei Schritt (a) das ursprüngliche Gemisch folgendes ist:

  . Lösemittel: 15,704 g 2-Methoxy-Ethanol
  . razemisches Gemisch: 4,296 g
  . (+)Antipode: 0,229 g

  und

  . $T_L = 37°C$
  . $T_B = 40°C$
  . Dauer der $T_B$-Stufe = 30 min

- bei Schritt (b) das Programmiergesetz zur Temperatursenkung von $T_B$ auf $T_F$ durch die nachstehenden zeitabhängigen Temperaturwerte gegeben ist:

| T (°C) | 40 | 35 | 30 | 25 | 20 | 20 |
|--------|----|----|----|----|----|----|
| t (min) | 0 | 12 | 22 | 30 | 38 | 46 |

- bei Schritt (c) für den verwendeten Versuchsaufbau das Rühren am Anfang 100 Umdrehungen/min und am Ende der Kristallisierung 150 Umdrehungen/min beträgt.
- bei Schritt (d) die Ausbeute der Kristalle auf Sinterglas Nr. 3 vorgenommen wird.
- bei Schritt (e) razemisches Gemisch hinzugefügt wird, die üblichen Ausgleiche vorgenommen werden, und:

  . $T_L = 37°C$
  . $T_B = 40°C$
  . Dauer der $T_B$-Stufe = 30 min

- bei Schritt (f) das Programmiergesetz zur Temperatursenkung von $T_B$ auf $T_F$ durch die nachstehenden zeitabhängigen Temperaturwerte gegeben ist:

| T (°C) | 40 | 35 | 30 | 25 | 20 | 20 |
|--------|----|----|----|----|----|----|
| t (min) | 0 | 12 | 22 | 30 | 38 | 46 |

- bei Schritt (g) für den verwendeten Versuchsaufbau das Rühren am Anfang 100 Umdrehungen/min und am Ende der Kristallisierung 150 Umdrehungen/min beträgt.
- bei Schritt (h) die Ausbeute der Kristalle auf Sinterglas Nr. 3 vorgenommen wird.
- bei Schritt (i) razemisches Gemisch hinzugefügt wird und die nötigen Ausgleiche vorgenommen werden.

12. Verfahren zur Spaltung der beiden optischen Enantiomere von 5-Methyl-5-(4-Methylphyenyl)-Hydantoin nach den Patentansprüchen 1 bis 9, dadurch gekennzeichnet, daß:

- bei Schritt (a) das ursprüngliche Gemisch folgendes ist:

  . Lösemittel: 27,68 g 2-Methoxy-Ethanol
  . razemisches Gemisch: 5,667 g
  . (+)Antipode: 0,458 g

  und

- $T_L = 39°C$
- $T_B = 41°C$
- Dauer der $T_B$-Stufe = 30 min

- bei Schritt (b) das Programmiergesetz zur Temperatursenkung von $T_B$ auf $T_F$ durch die nachstehenden zeitabhängigen Temperaturwerte gegeben ist:

| T (°C) | 41 | 31 | 31 | 21 | 21 | 14 |
|---|---|---|---|---|---|---|
| t (min) | 0 | 15 | 30 | 45 | 50 | 60 |

- bei Schritt (c) für den verwendeten Versuchsaufbau das Rühren am Anfang 150 Umdrehungen/min und am Ende der Kristallisierung 200 Umdrehungen/min beträgt.
- bei Schritt (d) die Ausbeute der Kristalle auf Sinterglas Nr. 3 vorgenommen wird.
- bei Schritt (e) razemisches Gemisch hinzugefügt wird, die üblichen Ausgleiche vorgenommen werden, und:

- $T_L = 39°C$
- $T_B = 41°C$
- Dauer der $T_B$-Stufe = 30 min

- bei Schritt (f) das Programmiergesetz zur Temperatursenkung von $T_B$ auf $T_F$ durch die nachstehenden zeitabhängigen Temperaturwerte gegeben ist:

| T (°C) | 41 | 31 | 31 | 21 | 21 | 14 |
|---|---|---|---|---|---|---|
| t (min) | 0 | 15 | 30 | 45 | 50 | 60 |

- bei Schritt (g) für den verwendeten Versuchsaufbau das Rühren am Anfang 150 Umdrehungen/min und am Ende der Kristallisierung 200 Umdrehungen/min beträgt.
- bei Schritt (h) die Ausbeute der Kristalle auf Sinterglas Nr. 3 vorgenommen wird.
- bei Schritt (i) razemisches Gemisch hinzugefügt wird, und die nötigen Ausgleiche vorgenommen werden.

13. Verfahren zur Spaltung der beiden optischen Enantiomere von 5-Ethyl-5-Phenyl-Hydantoin nach den Patentansprüchen 1 bis 9, dadurch gekennzeichnet, daß:

- bei Schritt (a) das ursprüngliche Gemisch folgendes ist:

- Lösemittel: 27,666 g 2-Methoxy-Ethanol
- razemisches Gemisch: 5,666 g
- (+)Antipode: 0,3877 g

und

- $T_L = 41°C$
- $T_B = 44,5°C$
- Dauer der $T_B$-Stufe = 30 min

- bei Schritt (b) das Programmiergesetz zur Temperatursenkung von $T_B$ auf $T_F$ durch die nachstehenden zeitabhängigen Temperaturwerte gegeben ist:

| T (°C) | 44,5 | 41 | 39 | 37 | 35 | 33 | 31 | 29 | 27 | 25 | 23 |
|---|---|---|---|---|---|---|---|---|---|---|---|
| t (min) | 0 | 10 | 15 | 20 | 25 | 30 | 35 | 41 | 46 | 51 | 56 |

- bei Schritt (c) für den verwendeten Versuchsaufbau das Rühren am Anfang 200 Umdrehungen/min und am Ende der Kristallisierung 275 Umdrehungen/min beträgt.
- bei Schritt (d) die Ausbeute der Kristalle auf Sinterglas Nr. 3 vorgenommen wird.
- bei Schritt (e) razemisches Gemisch hinzugefügt wird, die üblichen Ausgleiche vorgenommen werden, und:

- $T_L = 41°C$
- $T_B = 44,5°C$
- Dauer der $T_B$-Stufe = 30 min

- bei Schritt (f) das Programmiergesetz zur Temperatursenkung von $T_B$ auf $T_F$ durch die nachstehenden zeitabhängigen Temperaturwerte gegeben ist:

| T (°C) | 44,5 | 41 | 39 | 37 | 35 | 33 | 31 | 29 | 27 | 25 | 23 |
|--------|------|----|----|----|----|----|----|----|----|----|----|
| t (min) | 0 | 10 | 15 | 20 | 25 | 30 | 35 | 41 | 46 | 51 | 56 |

- bei Schritt (g) für den verwendeten Versuchsaufbau das Rühren am Anfang 200 Umdrehungen/min und am Ende der Kristallisierung 275 Umdrehungen/min beträgt.
- bei Schritt (h) die Ausbeute der Kristalle auf Sinterglas Nr. 3 vorgenommen wird.
- bei Schritt (i) razemisches Gemisch hinzugefügt wird und die nötigen Ausgleiche vorgenommen werden.

14. Verfahren zur Spaltung der beiden optischen Enantiomere von 5-Methyl-5-(4-Chlorophenyl)-Hydantoin nach den Patentansprüchen 1 bis 9, dadurch gekennzeichnet, daß:

- bei Schritt (a) das ursprüngliche Gemisch folgendes ist:

- Lösemittel: 95,7 g 2-Methoxy-Ethanol
- razemisches Gemisch: 6,166 g
- (+)Antipode: 0,430 g

und

- $T_L = 55°C$
- $T_B = 57°C$
- Dauer der $T_B$-Stufe = 60 min

- bei Schritt (b) das Programmiergesetz zur Temperatursenkung von $T_B$ auf $T_F$ durch die nachstehenden zeitabhängigen Temperaturwerte gegeben ist:

| T (°C) | 57 | 35 | 27 | 27 |
|--------|----|----|----|-----|
| t (min) | 0 | 32 | 52 | 115 |

- bei Schritt (c) für den verwendeten Versuchsaufbau das Rühren am Anfang 150 Umdrehungen/min und am Ende der Kristallisierung 200 Umdrehungen/min beträgt.
- bei Schritt (d) die Ausbeute der Kristalle auf Sinterglas Nr. 3 vorgenommen wird.
- bei Schritt (e) razemisches Gemisch hinzugefügt wird, die üblichen Ausgleiche vorgenommen werden, und:

- $T_L = 55°C$
- $T_B = 57°C$
- Dauer der $T_B$-Stufe = 60 min

- bei Schritt (f) das Programmiergesetz zur Temperatursenkung von $T_B$ auf $T_F$ durch die nachstehenden zeitabhängigen Temperaturwerte gegeben ist:

| T (°C) | 57 | 35 | 27 | 27 |
|--------|----|----|----|-----|
| t (min) | 0 | 32 | 52 | 115 |

- bei Schritt (g) für den verwendeten Versuchsaufbau das Rühren am Anfang 200 Umdrehungen/min und am Ende der Kristallisierung 275 Umdrehungen/min beträgt.
- bei Schritt (h) die Ausbeute der Kristalle auf Sinterglas Nr. 3 vorgenommen wird.
- bei Schritt (i) razemisches Gemisch hinzugefügt wird, und die nötigen Ausgleiche vorgenommen werden.

**15.** Verfahren zur Spaltung der beiden optischen Enantiomere von Threitol nach den Patentansprüchen 1 bis 9, dadurch gekennzeichnet, daß:

- bei Schritt (a) das ursprüngliche Gemisch folgendes ist:

  . Lösemittel: 87,5 g Ethanolgemisch 95%/ Wasser 5%
  . razemisches Gemisch: 12,50 g
  . (+) Antipode: 0,800 g

  und

  . $T_L = 27°C$
  . $T_B = 27,5°C$
  . Dauer der $T_B$-Stufe = 20 min

- bei Schritt (b) das Programmiergesetz zur Temperatursenkung von $T_B$ auf $T_F$ durch die nachstehenden zeitabhängigen Temperaturwerte gegeben ist:

| T (°C) | 27,5 | 26,8 | 26,8 | 23,3 | 23,3 | 19,4 | 19,4 |
|--------|------|------|------|------|------|------|------|
| t (min) | 0 | 1,5 | 5 | 11 | 15 | 22 | 60 |

- bei Schritt (c) für den verwendeten Versuchsaufbau das Rühren am Anfang 100 Umdrehungen/min und am Ende der Kristallisierung 120 Umdrehungen/min beträgt.
- bei Schritt (d) die Ausbeute der Kristalle auf Sinterglas Nr. 2 vorgenommen wird, worauf eine Spülung mit Diisopropylether erfolgt;
- bei Schritt (e) razemisches Gemisch hinzugefügt wird, die üblichen Ausgleiche vorgenommen werden, und:

  . $T_L = 27°C$
  . $T_B = 27,5°C$
  . Dauer der $T_B$-Stufe = 20 min

- bei Schritt (f) das Programmiergesetz zur Temperatursenkung von $T_B$ auf $T_F$ durch die nachstehenden zeitabhängigen Temperaturwerte gegeben ist:

| T (°C) | 27,5 | 26,8 | 26,8 | 23,3 | 23,3 | 19,4 | 19,4 |
|--------|------|------|------|------|------|------|------|
| t (min) | 0 | 1,5 | 5 | 11 | 15 | 22 | 60 |

- bei Schritt (g) für den verwendeten Versuchsaufbau das Rühren am Anfang 100 Umdrehungen/min und am Ende der Kristallisierung 120 Umdrehungen/min beträgt.
- bei Schritt (h) die Ausbeute der Kristalle auf Sinterglas Nr. 2 vorgenommen wird, worauf eine Spülung mit Diisopropylether erfolgt;
- bei Schritt (i) razemisches Gemisch hinzugefügt wird und die nötigen Ausgleiche vorgenommen werden.

**Claims**

**1.** Process for splitting-off two optical enantiomers by preferential crystallization, characterized in that it comprises the following steps:

a) an assembly is made composed of a racemic mix of crystals in conglomerate form, the first enantiomer and the solvent, for which the figurative point E defined by the concentration and temperature variables TB are located in the two-phase domain of the first enantiomer in excess, and is in equilibrium with its saturated solution;
b) a programming law is applied to lower the temperature of the two-phase mix prepared in step (a), the said programming law being such that the parent solutions keep a low supersaturation that gives priority to growth of the enantiomer present in crystal form, while preventing spontaneous nucleation of the second enantiomer present in the solution;

c) throughout the period of crystalline growth in step b), a slowly increasing stirring speed is adapted as a function of time such that at all times it is sufficiently slow to favor growth of the first enantiomer while avoiding generating excessive striction forces causing uncontrolled nucleation, and sufficiently fast to achieve a homogeneous suspension and fast renewal of the parent solution around each crystallite in the first enantiomer;

d) the crystals in the first enantiomer are collected;

e) the racemic mix of crystals in conglomerate form is added to the parent solutions resulting from the collection done in step (d), and the temperature of the new mix is increased to a constant temperature $T_B$ for the time necessary to achieve a thermodynamic equilibrium such that the figurative point E' is symmetric with E about the plane of racemic mixes in the solvent, (-) antipode, (+) antipode, system the said point E' being located in the two-phase domain of the second enantiomer in excess and in equilibrium with its saturated solution;

f) the same programming law as in step (b) is applied to lower the temperature of the two-phase mix prepared in step (e) containing the second enantiomer, such that the parent solutions keep a low supersaturation during crystallization to give priority to growth of the enantiomer present in crystal form, while preventing spontaneous nucleation of the first enantiomer present in the solution;

g) throughout the period of crystalline growth in step f), a slowly increasing stirring speed is adapted as a function of time such that at all times it is sufficiently slow to favor growth of the second enantiomer while avoiding generating excessive striction forces causing uncontrolled nucleation, and sufficiently fast to achieve a homogeneous suspension and fast renewal of the parent solution around each crystallite in the second enantiomer;

h) the crystals in the second enantiomer are collected;

i) the racemic mix of crystals in conglomerate form is added to the parent solutions resulting from the crystalline collection done in step (h), to obtain an assembly with exactly the same composition as the initial assembly E;

j) steps (a), (b), (c), (d), (e), (f), (g), (h) and (i) are repeated to obtain the first and then the second enantiomers respectively.

2. Process according to claim 1, characterized in that the choice of the solvent(s) and the working temperature range in step (a) are defined so as to simultaneously obtain:

- antipodes that make a conglomerate and for which the racemic compound, if any, is metastable within the working temperature range;
- sufficiently concentrated solutions with low viscosity and low vapor pressure;
- a lack of solvolysis and racemization;
- stability of the solvates if they are present in equilibrium and if they are splittable enantiomers.

3. Process according to either of claims 1 and 2, characterized in that in steps (a) and (e), the temperature $T_B$ exceeds the homogenization temperature $T_L$ to homogenize the quantity of racemic mix contained in the initial suspension and in that, starting from the curve of the variation of $T_{HOMO}$ as a function of the enantiomeric excess and for a constant concentration of the racemic mix $X_L$, the said temperature $T_B$ is defined such that the mass of fine crystals in the first enantiomer in steps (a) and (i) and the second enantiomer in step (e), in equilibrium with their saturated solution, represents a maximum of 50% and preferably between approximately 25 and 40% of the expected collection.

4. Process according to any one of the previous claims, characterized in that in steps (b) and (f), the programming law for lowering the temperature from $T_B$ to TF adapted to the experimental setup is defined so as to:

- obtain a low supersaturation throughout the crystallization period of the enantiomer present in the form of crystals at the beginning of each cycle, this low supersaturation causing slow secondary growth and nucleation;
- reach the maximum supersaturation of the other enantiomer without primary nucleation at $T_F$,
- obtain a collection in crystals in steps (d) and (h) which, after addition of a racemic mix and compensation in steps (e) and (i), enables cyclic operations.

5. Process according to claim 4, characterized in that the programming law to lower the temperature is determined in its part between $T_L$ and $T_F$ by cooling the concentration solution $X_L$ from $T_L$ +1°C to $T_F$, where $T_F$ is less than $T_L - (T_{HOMO} - T_L)$, in order to obtain a supersaturated solution without primary nucleation, while enabling double collection of the initial enantiomeric excess, and in that the said programming law to lower the temperature is determined in its part between $T_B$ and $T_L$, by extrapolation of this same law determined between $T_L$ + 1°C and $T_F$.

**6.** Process according to any one of the previous claims, characterized in that in steps (b) and (f), the thermicity accompanying the deposit of the first enantiomer and the second enantiomer is integrated in the temperature reduction programming law.

**7.** Process according to any one of the previous claims, characterized in that compensations in solvent are done in steps (e) and (i).

**8.** Process according to any one of the previous steps, characterized in that in steps (a), (e) and (i), the fine crystals in the racemic mix that are added in conglomerate form, were subjected to a prior treatment such as grinding and sieving, treatment by ultrasound waves, partial freeze dying before they were added, to accelerate the solution step.

**9.** Process according to any one of the previous claims, characterized in that the stirring speed is increased during steps (a), (e) and (i).

**10.** Process for splitting-off two optical enantiomers of double sodium tartrate and tetrahydrated ammonium according to claims 1 to 9, characterized in that:

- in step (a), the initial mix is as follows:

  - solvent: 5.67 g of water
  - racemic mix: 4.83 g
  - (+) antipode: 0.23 g, and
  - $T_L = 16.3°C$,
  - $T_B = 17.3°C$
  - time for which the temperature is kept at $T_B = 40$ min.

- in step (b), the programming law for the temperature drop from $T_B$ to $T_F$ is given by temperature values that depend on the time as shown below:

| T (°C) | 17.3 | 17.0 | 16.5 | 15.4 | 14.0 | 13.0 | 12.1 | 12.0 | 12.0 |
|--------|------|------|------|------|------|------|------|------|------|
| t (min.) | 0 | 3 | 5 | 8 | 12.5 | 16 | 20 | 25 | 30 |

- in step (c), in the experimental setup used, stirring takes place at 150 rpm at the beginning and 170 rpm at the end of crystallization;
- in step (d), crystals are collected on sintered glass No. 2;
- in step (e), the ground racemic mix sieved to 250 µ is added, and ammonia is added in addition to normal compensations for losses, to maintain a slight ammonia excess and:

  - $T_L = 16.3°C$,
  - $T_B = 17.3°C$
  - time for which the temperature is kept at $T_B = 40$ min.

- in step (f), the programming law for the temperature drop from $T_B$ to $T_F$ is given by temperature values that depend on the time as shown below:

| T (°C) | 17.3 | 17.0 | 16.5 | 15.4 | 14.0 | 13.0 | 12.1 | 12.0 | 12.0 |
|--------|------|------|------|------|------|------|------|------|------|
| t (mn) | 0 | 3 | 5 | 8 | 12.5 | 16 | 20 | 25 | 30 |

- in step (g), in the experimental setup used, stirring takes place at 150 rpm at the beginning and 170 rpm at the end of crystallization.
- in step (h), crystals are collected on sintered glass No. 2.
- in step (i), the ground racemic mix sieved to 250 µ is added, and ammonia is added in addition to normal compensations for losses, to maintain a slight ammonia excess.

**11.** Process for splitting-off the two optical enantiomers of 5-methyl-5-phenyl-hydantoin according to claims 1 to 9, characterized in that:

- in step (a), the initial mix is as follows:

  - solvent: 15.704 g of 2-methoxy-ethanol
  - racemic mix: 4.296 g
  - (+) antipode: 0.229 g,

  and

  - $T_L = 37°C$,
  - $T_B = 40°C$
  - time for which the temperature is kept at $T_B = 30$ min.

- in step (b), the programming law for the temperature drop from $T_B$ to $T_F$ is given by temperature values that depend on the time as described below:

| T (°C)   | 40 | 35 | 30 | 25 | 20 | 20 |
|----------|----|----|----|----|----|----|
| t (min.) | 0  | 12 | 22 | 30 | 38 | 46 |

- in step (c), in the experimental setup used, stirring takes place at 100 rpm at the beginning and 150 rpm at the end of crystallization;
- in step (d), crystals are collected on sintered glass No. 3;
- in step (e), the racemic mix is added, the normal compensations are made and:

  - $T_L = 37°C$,
  - $T_B = 40°C$
  - time for which the temperature is kept at $T_B = 30$ min.

- in step (f), the programming law for the temperature drop from $T_B$ to $T_F$ is given by temperature values that depend on the time as described below:

| T (°C)   | 40 | 35 | 30 | 25 | 20 | 20 |
|----------|----|----|----|----|----|----|
| t (min.) | 0  | 12 | 22 | 30 | 38 | 46 |

- in step (g), in the experimental setup used, stirring takes place at 100 rpm at the beginning and 150 rpm at the end of crystallization.
- in step (h), crystals are collected on sintered glass No. 3.
- in step (i), the racemic mix is added and the normal compensations are made.

12. Process for splitting-off the two optical enantiomers of 5-methyl-5-(4-methyl-phenyl)-hydantoin according to claims 1 to 9, characterized in that:

- in step (a), the initial mixture is as follows:

  - solvent: 27.68 g of 2-methoxy-ethanol
  - racemic mix: 5.667 g
  - (+) antipode: 0.458 g,

  and

  - $T_L = 39°C$,
  - $T_B = 41°C$
  - time for which the temperature is kept at $T_B = 30$ min.

- in step (b), the programming law for the temperature drop from $T_B$ to $T_F$ is given by temperature values that depend on the time as described below:

| T (°C) | 41 | 31 | 31 | 21 | 21 | 14 |
|--------|----|----|----|----|----|----|
| t (min.) | 0 | 15 | 30 | 45 | 50 | 60 |

- in step (c), in the experimental setup used, stirring takes place at 150 rpm at the beginning and 200 rpm at the end of crystallization;
- in step (d), crystals are collected on sintered glass No. 3;
- in step (e), the racemic mix is added, the normal compensations are made and:

  - $T_L = 39°C$,
  - $T_B = 41°C$
  - time for which the temperature is kept at $T_B = 30$ min.

- in step (f) , the programming law for the temperature drop from $T_B$ to $T_F$ is given by temperature values that depend on the time as described below:

| T (°C) | 41 | 31 | 31 | 21 | 21 | 14 |
|--------|----|----|----|----|----|----|
| t (min.) | 0 | 15 | 30 | 45 | 50 | 60 |

- in step (g), in the experimental setup used, stirring takes place at 150 rpm at the beginning and 200 rpm at the end of crystallization.
- in step (h), crystals are collected on sintered glass No. 3.
- in step (i), the racemic mix is added and the normal compensations are made.

13. Process for splitting-off the two optical enantiomers of 5-ethyl-5-phenyl-hydantoin according to claims 1 to 9, characterized in that:

- in step (a), the initial mixture is as follows:

  - solvent: 27.666 g of 2-methoxy-ethanol
  - racemic mix: 5.666 g
  - (+) antipode: 0.3877 g, and
  - $T_L = 41°C$,
  - $T_B = 44.5°C$
  - time for which the temperature is kept at $T_B = 30$ min.

- in step (b), the programming law for the temperature drop from $T_B$ to $T_F$ is given by temperature values that depend on the time as described below:

| T (°C) | 44.5 | 41 | 39 | 37 | 35 | 33 | 31 | 29 | 27 | 25 | 23 |
|--------|------|----|----|----|----|----|----|----|----|----|----|
| t (min.) | 0 | 10 | 15 | 20 | 25 | 30 | 35 | 41 | 46 | 51 | 56 |

- in step (c), in the experimental setup used, stirring takes place at 200 rpm at the beginning and 275 rpm at the end of crystallization;
- in step (d), crystals are collected on sintered glass No. 3;
- in step (e), the racemic mix is added, the normal compensations are made and:

  - $T_L = 41°C$,
  - $T_B = 44.5°C$
  - time for which the temperature is kept at $T_B = 30$ min.

- in step (f), the programming law for the temperature drop from $T_B$ to $T_F$ is given by temperature values that depend on the time as described below:

| T (°C) | 44.5 | 41 | 39 | 37 | 35 | 33 | 31 | 29 | 27 | 25 | 23 |
|--------|------|----|----|----|----|----|----|----|----|----|----|

(continued)

| t (min.) | 0 | 10 | 15 | 20 | 25 | 30 | 35 | 41 | 46 | 51 | 56 |
|---|---|---|---|---|---|---|---|---|---|---|---|

- in step (g), in the experimental setup used, stirring takes place at 200 rpm at the beginning and 275 rpm at the end of crystallization;
- in step (h), crystals are collected on sintered glass No. 3;
- in step (i), the racemic mix is added and the necessary compensations are made.

14. Process for splitting-off the two optical enantiomers of 5-methyl-5-(4-chlorophenyl)-hydantoin according to claims 1 to 9, characterized in that:

- in step (a), the initial mixture is as follows:

  • solvent: 95.7 g of 2-methoxy-ethanol
  • racemic mix: 6.166 g
  • (+) antipode: 0.430 g,

  and

  • $T_L = 55°C$,
  • $T_B = 57°C$
  • time for which the temperature is kept at $T_B = 60$ min.

- in step (b), the programming law for the temperature drop from $T_B$ to $T_F$ is given by temperature values that depend on the time as described below:

| T (°C) | 57 | 35 | 27 | 27 |
|---|---|---|---|---|
| t (min.) | 0 | 32 | 52 | 115 |

- in step (c), in the experimental setup used, stirring takes place at 150 rpm at the beginning and 200 rpm at the end of crystallization;
- in step (d), crystals are collected on sintered glass No. 3;
- in step (e), the racemic mix is added, the normal compensations are made and:

  • $T_L = 55°C$,
  • $T_B = 57°C$
  • time for which the temperature is kept at $T_B = 60$ min.

- in step (f), the programming law for the temperature drop from $T_B$ to $T_F$ is given by temperature values that depend on the time as described below:

| T (°C) | 57 | 35 | 27 | 27 |
|---|---|---|---|---|
| t (min.) | 0 | 32 | 52 | 115 |

- in step (g), in the experimental setup used, stirring takes place at 200 rpm at the beginning and 275 rpm at the end of crystallization;
- in step (h), crystals are collected on sintered glass No. 3;
- in step (i), the racemic mix is added and the necessary compensations are made.

15. Process for splitting-off the two optical enantiomers of threitol according to claims 1 to 9, characterized in that:

- in step (a), the initial mixture is as follows:

  • solvent: 87.5 g of a 95% ethanol / 5% water mix
  • racemic mix: 12.50 g

- (+) antipode: 0.800 g,

and

- $T_L = 27°C$,
- $T_B = 27.5°C$
- time for which the temperature is kept at $T_B = 20$ min.

- in step (b), the programming law for the temperature drop from $T_B$ to $T_F$ is given by temperature values that depend on the time as described below:

| T (°C) | 27.5 | 26.8 | 26.8 | 23.3 | 23.3 | 19.4 | 19.4 |
|--------|------|------|------|------|------|------|------|
| t (min.) | 0 | 1.5 | 5 | 11 | 15 | 22 | 60 |

- in step (c), in the experimental setup used, stirring takes place at 100 rpm at the beginning and 120 rpm at the end of crystallization;
- in step (d), crystals are collected on sintered glass No. 2 followed by washing with diisopropylether;
- in step (e), the racemic mix is added, the normal compensations are made and:

- $T_L = 27°C$,
- $T_B = 27.5°C$
- time for which the temperature is kept at $T_B = 20$ min.

- in step (f), the programming law for the temperature drop from $T_B$ to $T_F$ is given by temperature values that depend on the time as described below:

| T (°C) | 27.5 | 26.8 | 26.8 | 23.3 | 23.3 | 19.4 | 19.4 |
|--------|------|------|------|------|------|------|------|
| t (min.) | 0 | 1.5 | 5 | 11 | 15 | 22 | 60 |

- in step (g), in the experimental setup used, stirring takes place at 100 rpm at the beginning and 120 rpm at the end of crystallization;
- in step (h), crystals are collected on sintered glass No. 2 followed by washing with diisopropylether;
- in step (i), the racemic mix is added and the necessary compensations are made.

**fig.1**

Légende : ——— équilibre à $T_F$
— — équilibre à $T_D$
— ·· coupe isoplèthe RY

**Fig 2**

**fig.3**

Fig 4

**Fig.5**

**Fig.6**

**Fig.7**

Fig.8